# EUROPEAN PATENT SPECIFICATION

(11) **EP 1 032 672 B1**
(45) Date of publication and mention of the grant of the patent: **31.12.2008**
(21) Application number: 98958067.5
(22) Date of filing: 18.11.1998
(51) Int. Cl.: C12N 15/28, C07K 14/525, A61K 38/19, C07K 19/00, C07K 16/24

(54) **DNA19355 POLYPEPTIDE, A TUMOR NECROSIS FACTOR HOMOLOG**
DNA 19355 POLYPEPTIDE, EIN HOMOLOG DES TUMORNEKROSEFAKTORS
POLYPEPTIDE ADN19355, HOMOLOGUE DU FACTEUR DE NECROSE DES TUMEURS

(30) Priority: 18.11.1997 US 65635 P; 12.12.1997 US 69661 P
(43) Date of publication of application: 06.09.2000
(73) Proprietor: Genentech, Inc., South San Francisco CA 94080-4990 (US)
(72) Inventor: ASHKENAZI, Avi, J., San Mateo, CA 94402 (US); GURNEY, Austin, L., Belmont, CA 94002 (US); MARSTERS, Scot, A., San Carlos, CA 94070 (US); PITTI, Robert, El Cerrito, CA 94530 (US); BAKER, Kevin, P., San Mateo, CA 94401 (US); GODOWSKI, Paul, J., Burlingame, CA 94010 (US); MARK, Melanie, R., Burlingame, CA 94010 (US)
(74) Representative: Kiddle, Simon John
(86) International application number: PCT/US1998/024621
(87) International publication number: WO 1999/025834

(56) References cited:
- WO-A-97/01633
- WO-A-98/07880
- GRUSS H.-J. AND DOWER S.K.: "Tumor necrosis factor ligand superfamily: involvement in the pathology of malignant lymphomas" BLOOD, vol. 85, no. 12, 15 June 1995, pages 3378-3404, XP002094502 cited in the application
- AGGARWAL B.B. AND NATARAJAN K.: "Tumor necrosis factor: developments during the last decade" EUROPEAN CYTOKINE NETWORK, vol. 7, no. 2, April 1996 - June 1996, pages 93-124, XP002094503
- GRUSS H.-J-: "Molecular, structural, and biological characteristics of the tumor necrosis factor ligand superfamily" INTERNATIONAL JOURNAL OF CLINICAL AND LABORATORY RESEARCH, vol. 26, no. 3, 1996, pages 143-159, XP002094504

### SEQUENCE LISTING

<110> GENENTECH INC.
<120> DNA19355 POLYPEPTIDE, A TUMOR NECROSIS FACTOR HOMOLOG
<130> 11669.26WO01
<140> NEW FILING
   <141> 1998-11-18
<150> 60/069,661
   <151> 1997-12-12
<150> 60/065,635
   <151> 1997-11-18
<160> 8
<170> PatentIn Ver. 2.0
<210> 1
   <211> 177
   <212> PRT
   <213> Homo sapiens
<400> 1
   Met Cys Leu Ser His Leu Glu Asn Met Pro Leu Ser His Ser Arg Thr
   1               5                   10                  15
   Gln Gly Ala Gln Arg Ser Ser Trp Lys Leu Trp Leu Phe Cys Ser Ile
               20                  25                  30
   Val Met Leu Leu Phe Leu Cys Ser Phe Ser Trp Leu Ile Phe Ile Phe
           35                  40                  45
   Leu Gln Leu Glu Thr Ala Lys Glu Pro Cys Met Ala Lys Phe Gly Pro
       50                  55                  60
   Leu Pro Ser Lys Trp Gln Met Ala Ser Ser Glu Pro Pro Cys Val Asn
   65                  70                  75                  80
   Lys Val Ser Asp Trp Lys Leu Glu Ile Leu Gln Asn Gly Leu Tyr Leu
                   85                  90                  95
   Ile Tyr Gly Gln Val Ala Pro Asn Ala Asn Tyr Asn Asp Val Ala Pro
               100                 105                 110
   Phe Glu Val Arg Leu Tyr Lys Asn Lys Asp Met Ile Gln Thr Leu Thr
           115                 120                 125
   Asn Lys Ser Lys Ile Gln Asn Val Gly Gly Thr Tyr Glu Leu His Val
       130                 135                 140
   Gly Asp Thr Ile Asp Leu Ile Phe Asn Ser Glu His Gln Val Leu Lys
   145                 150                 155                 160
   Asn Asn Thr Tyr Trp Gly Ile Ile Leu Leu Ala Asn Pro Gln Phe Ile
                   165                 170                 175
   Ser
<210> 2
   <211> 1964
   <212> DNA
   <213> Homo sapiens
<400> 2
   cagctctcat ttctccaaaa atgtgtttga gccacttgga aaatatgcct ttaagccatt 60
   caagaactca aggagctcag agatcatcct ggaagctgtg gctcttttgc tcaatagtta 120
   tgttgctatt tctttgctcc ttcagttggc taatctttat ttttctccaa ttagagactg 180
   ctaaggagcc ctgtatggct aagtttggac cattaccctc aaaatggcaa atggcatctt 240
   ctgaacctcc ttgcgtgaat aaggtgtctg actggaagct ggagatactt cagaatggct 300
   tatatttaat ttatggccaa gtggctccca atgcaaacta caatgatgta gctccttttg 360
   aggtgcggct gtataaaaac aaagacatga tacaaactct aacaaacaaa tctaaaatcc 420
   aaaatgtagg agggacttat gaattgcatg ttggggacac catagacttg atattcaact 480
   ctgagcatca ggttctaaaa aataatacat actggggtat cattttacta gcaaatcccc 540
   aattcatctc ctagagactt gatttgatct cctcattccc ttcagcacat gtagaggtgc 600
   cagtgggtgg attggaggga gaagatattc aatttctaga gtttgtctgt ctacaaaaat 660
   caacacaaac agaactcctc tgcacgtgaa ttttcatcta tcatgcctat ctgaaagaga 720
   ctcaggggaa gagccaaaga cttttggttg gatctgcaga aatacttcat taatccatga 780
   taaaacaaat atggatgaca gaggacatgt gcttttcaaa gaatctttat ctaattcttg 840
   aattcatgag tggaaaaatg gagttctatt cccatggaag atttacctgg tatgcaaaaa 900
   ggatctgggg cagtagcctg gctttgttct catattcttg ggctgctgta attcattctt 960
   ctcatactcc catcttctga gaccctccca ataaaaagta gactgatagg atggccacag 1020
   atatgcctac cataccctac tttagatatg gtggtgttag aagataaaga acaatctgag 1080
   aactattgga atagaggtac aagtggcata aaatggaatg tacgctatct ggaaatttct 1140
   cttggtttta tcttcctcag gatgcagggt gctttaaaaa gccttatcaa aggagtcatt 1200
   ccgaaccctc acgtagagct ttgtgagacc ttactgttgg tgtgtgtgtc taaacattgc 1260
   taattgtaaa gaaagagtaa ccattagtaa tcattaggtt taaccccaga atggtattat 1320
   cattactgga ttatgtcatg taatgattta gtatttttag ctagctttcc acagtttgca 1380
   aagtgctttc gtaaaacagt tagcaattct atgaagttaa ttgggcaggc atttggggga 1440
   aaattttagt gatgagaatg tgatagcata gcatagccaa ctttcctcaa ctcataggac 1500
   aagtgactac aagaggcaat gggtagtccc ctgcattgca ctgtctcagc tttagaattg 1560
   ttatttctgc tatcgtgtta taagactcta aaacttagcg aattcacttt tcaggaagca 1620
   tattcccctt tagcccaagg tgagcagagt gaagctacaa cagatctttc ctttaccagc 1680
   acactttttt ttttttttcc tgcctgaatc agggagatcc aggatgctgt tcaggccaaa 1740
   tcccaaccaa attccccttt tcactttgca gggcccatct tagtcaaatg tgctaacttc 1800
   taaaataata aatagcacta attcaaaatt tttggaatct taaattagct acttgcnggt 1860
   tgcttgttga aaggnatata atgattacat tgtaaacaaa tttaaaatat ttatggatat 1920
   ttgtgaaaag ctgcattatg ttaaataata ttacatgtaa agct                  1964
<210> 3
   <211> 38
   <212> DNA
   <213> Unknown
<220>
   <223> Description of Unknown Organism:Unknown
<400> 3
   gacgacaagc atatgttaga gactgctaag gagccctg 38
<210> 4
   <211> 34
   <212> DNA
   <213> Unknown
<220>
   <223> Description of Unknown Organism:Unknown
<400> 4
   tagcagccgg atcctaggag atgaattggg gatt 34
<210> 5
   <211> 24
   <212> PRT
   <213> Unknown
<220>
   <223> Description of Unknown Organism:Unknown
<400> 5
   Met Gly His His His His His His His His His His Ser Ser Gly His
     1               5                  10                  15
   Ile Asp Asp Asp Asp Lys His Met
                20
<210> 6
   <211> 29
   <212> DNA
   <213> Unknown
<220>
   <223> Description of Unknown Organism:Unknown
<400> 6
   atcagggact ttccgctggg gactttccg 29
<210> 7
   <211> 42
   <212> DNA
   <213> Unknown
<220>
   <223> Description of Unknown Organism:Unknown
<400> 7
   tgtaaaacga cggccagttt ctctcagaga aacaagcaaa ac 42
<210> 8
   <211> 43
   <212> DNA
   <213> Unknown
<220>
   <223> Description of Unknown Organism:Unknown
<400> 8
   caggaaacag ctatgaccga agtggaccaa aggtctatcg cta 43

## Description

### FIELD OF THE INVENTION

The present invention relates generally to the identification and isolation of novel DNA and to the recombinant production of novel polypeptides, designated herein as "DNA19355".

### BACKGROUND OF THE INVENTION

Control of cell numbers in mammals is believed to be determined, in part, by a balance between cell proliferation and cell death. One form of cell death, sometimes referred to as necrotic cell death, is typically characterized as a pathologic form of cell death resulting from some trauma or cellular injury. In contrast, there is another, "physiologic" form of cell death which usually proceeds in an orderly or controlled manner. This orderly or controlled form of cell death is often referred to as "apoptosis" [see, *e.g.*, Barr et al., Bio/Technology, 12:487-493 (1994); Steller et al., Science, 267:1445-1449 (1995)]. Apoptotic cell death naturally occurs in many physiological processes, including embryonic development and clonal selection in the immune system [Itoh et al., Cell, 66:233-243 (1991)]. Decreased levels of apoptotic cell death have been associated with a variety of pathological conditions, including cancer, lupus, and herpes virus infection [Thompson, Science, 267:1456-1462 (1995)]. Increased levels of apoptotic cell death may be associated with a variety of other pathological conditions, including AIDS, Alzheimer's disease, Parkinson's disease, amyotrophic lateral sclerosis, multiple sclerosis, retinitis pigmentosa, cerebellar degeneration, aplastic anemia, myocardial infarction, stroke, reperfusion injury, and toxin-induced liver disease [see, Thompson, supra].

Apoptotic cell death is typically accompanied by one or more characteristic morphological and biochemical changes in cells, such as condensation of cytoplasm, loss of plasma membrane microvilli, segmentation of the nucleus, degradation of chromosomal DNA or loss of mitochondrial function. A variety of extrinsic and intrinsic signals are believed to trigger or induce such morphological and biochemical cellular changes [Raff, Nature, 356:397-400 (1992); Steller, supra; Sachs et al., Blood, 82:15 (1993)]. For instance, they can be triggered by hormonal stimuli, such as glucocorticoid hormones for immature thymocytes, as well as withdrawal of certain growth factors [watanabe-Fukunaga et al., Nature, 356:314-317 (1992)]. Also, some identified oncogenes such as *myc, rel*, and *E1A,* and tumor suppressors, like *p53*, have been reported to have a role in inducing apoptosis. Certain chemotherapy drugs and some forms of radiation have likewise been observed to have apoptosis-inducing activity [Thompson, supra].

Various molecules, such as tumor necrosis factor-α ("TNF-α"), tumor necrosis factor-β ("TNF-β" or "lymphotoxin-α"), lymphotoxin-β ("LT-β"), CD30 ligand, CD27 ligand, CD40 ligand, OX-40 ligand, 4-1BB ligand, Apo-1 ligand (also referred to as Fas ligand or CD95 ligand), and Apo-2 ligand (also referred to as TRAIL) have been identified as members of the tumor necrosis factor ("TNF") family of cytokines [See, e.g., Gruss and Dower, Blood, 85:3378-3404 (1995); Pitti et al., J. Biol. Chem., 271:12687-12690 (1996); Wiley et al., Immunity, 3:673-682 (1995); Browning et al., Cell, 72:847-856 (1993); Armitage et al. Nature, 357:80-82 (1992)]. Among these molecules, TNF-α, TNFR-β, CD30 ligand, 4-1BB ligand, Apo-1 ligand, and Apo-2 ligand (TRAIL) have been reported to be involved in apoptotic cell death. Both TNF-α and TNF-β have been reported to induce apoptotic death in susceptible tumor cells [Schmid et al., Proc. Natl. Acad. Sci., 83:1881 (1986); Dealtry et al., Eur. J. Immunol., 17:689 (1987)]. Zheng et al. have reported that TNF-α is involved in post-stimulation apoptosis of CD8-positive T cells [Zheng et al., Nature, 377:348-351 (1995)]. Other investigators have reported that CD30 ligand may be involved in deletion of self-reactive T cells in the thymus [Amakawa et al., Cold Spring Harbor Laboratory Symposium on Programmed Cell Death, Abstr. No. 10, (1995)].

Mutations in the mouse Fas/Apo-1 receptor or ligand genes (called Ipr and gld, respectively) have been associated with some autoimmune disorders, indicating that Apo-1 ligand may play a role in regulating the clonal deletion of self-reactive lymphocytes in the periphery [Krammer et al., Curr. Op. Immunol., 6:279-289 (1994); Nagata et al., Science, 267:1449-1456 (1995)]. Apo-1 ligand is also reported to induce post-stimulation apoptosis in CD4-positive T lymphocytes and in B lymphocytes, and may be involved in the elimination of activated lymphocytes when their function is no longer needed [Krammer et al., supra; Nagata et al., supra]. Agonist mouse monoclonal antibodies specifically binding to the Apo-1 receptor have been reported to exhibit cell killing activity that is comparable to or similar to that of TNF-α [Yonehara et al., J. Exp. Med., 169:1747-1756 (1989)].

Induction of various cellular responses mediated by such TNF family cytokines is believed to be initiated by their binding to specific cell receptors. Two distinct TNF receptors of approximately 55-kDa (TNFR1) and 75-kDa (TNFR2) have been identified [Hohman et al., J. Biol. Chem., 264:14927-14934 (1989); Brockhaus et al., Proc. Natl. Acad. Sci., 87:3127-3131 (1990); EP 417,563, published March 20, 1991] and human and mouse cDNAs corresponding to both receptor types have been isolated and characterized [Loetscher et al., Cell, 61:351 (1990); Schall et al., Cell, 61:361 (1990); Smith et al., Science, 248:1019-1023 (1990); Lewis et al., Proc. Natl. Acad. Sci., 88:2830-2834 (1991); Goodwin et al., Mol. Cell. Biol., 11:3020-3026 (1991)]. Extensive polymorphisms have been associated with both TNF receptor genes [see, e.g., Takao et al., Immunogenetics, 37:199-203 (1993)]. Both TNFRs share the typical structure of cell surface receptors including extracellular, transmembrane and intracellular regions. The extracellular portions of both receptors are found naturally also as soluble TNF-binding proteins [Nophar, Y. et al., EMBO J., 9:3269 (1990); and Kohno, T. et al., Proc. Natl. Acad. Sci. U.S.A., 87:8331 (1990)]. More recently, the cloning of recombinant soluble TNF receptors was reported by Hale et al. [J. Cell.. Biochem. Supplement 15F, 1991, p. 113 (P424)].

The extracellular portion of type 1 and type 2 TNFRs (TNFR1 and TNFR2) contains a repetitive amino acid sequence pattern of four cysteine-rich domains (CRDs) designated 1 through 4, starting from the NH,-terminus. Each CRD is about 40 amino acids long and contains 4 to 6 cysteine residues at positions which are well conserved [Schall et al., supra; Loetscher et al., supra; Smith et al., supra; Nophar et al., supra; Kohno et al., supra]. In TNFR1, the approximate boundaries of the four CRDs are as follows: CRD1- amino acids 14 to about 53; CRD2- amino acids from about 54 to about 97; CRD3- amino acids from about 98 to about 138; CRD4- amino acids from about 139 to about 167. In TNFR2, CRD1 includes amino acids 17 to about 54; CRD2- amino acids from about 55 to about 97; CRD3- amino acids from about 98 to about 140; and CRD4- amino acids from about 141 to about 179 [Banner et al., Cell, 73:431-435 (1993)]. The potential role of the CRDs in ligand binding is also described by Banner et al., supra.

A similar repetitive pattern of CRDs exists in several other cell-surface proteins, including the p75 nerve growth factor receptor (NGFR) [Johnson et al., Cell, 47:545 (1986); Radeke et al., Nature, 325:593 (1987)], the B cell antigen CD40 [Stamenkovic et al., EMBO J., 8:1403 (1989)], the T cell antigen OX40 [Mallet et al., EMBO J., 9:1063 (1990)] and the Fas antigen [Yonehara et al., supra and Itoh et al., Cell, 66:233-243 (1991)]. CRDs are also found in the soluble TNFR (sTNFP)-like T2 proteins of the Shope and myxoma poxviruses [Upton et al., Virology, 160:20-29 (1987); Smith et al., Biochem. Biophys. Res. Commun., 176:335 (1991); Upton et al., Virology, 184:370 (1991)]. Optimal alignment of these sequences indicates that the positions of the cysteine residues are well conserved. These receptors are sometimes collectively referred to as members of the TNF/NGF receptor superfamily. Recent studies on p75NGFR showed that the deletion of CRD1 [Welcher, A.A. et al., Proc. Natl. Acad. Sci. USA, 88:159-163 (1991)] or a 5-amino acid insertion in this domain [Yan, H. and Chao, M.V., J. Biol. Chem., 266:12099-12104 (1991)] had little or no effect on NGF binding [Yan, H. and Chao, M.V., supra]. p75 NGFR contains a proline-rich stretch of about 60 amino acids, between its CRD4 and transmembrane region, which is not involved in NGF binding [Peetre, C. et al., Eur. J. Hematol., 41:414-419 (1988); Seckinger, P. et al., J. Biol. Chem., 264:11966-11973 (1989); Yan, H. and Chao, M.V., supra]. A similar proline-rich region is found in TNFR2 but not in TNFR1.

Itoh et al. disclose that the Apo-1 receptor can signal an apoptotic cell death similar to that signaled by the 55-kDa TNFR1 [Itoh et al., supra]. Expression of the Apo-1 antigen has also been reported to be down-regulated along with that of TNFR1 when cells are treated with either TNF-α or anti-Apo-1 mouse monoclonal antibody [Krammer et al., supra; Nagata et al., supra] Accordingly, some investigators have hypothesized that cell lines that co-express both Apo-1 and TNFR1 receptors may mediate cell killing through common signaling pathways [Id.].

The TNF family ligands identified to date, with the exception of lymphotoxin-α, are type II transmembrane proteins, whose C-terminus is extracellular. In contrast, most receptors in the TNF receptor (TNFR) family identified to date are type I transmembrane proteins. In both the TNF ligand and receptor families, however, homology identified between family members has been found mainly in the extracellular domain ("ECD"). Several of the TNF family cytokines, including TNF-α, Apo-1 ligand and CD40 ligand, are cleaved proteolytically at the cell surface; the resulting protein in each case typically forms a homotrimeric molecule that functions as a soluble cytokine. TNF receptor family proteins are also usually cleaved proteolytically to release soluble receptor ECDs that can function as inhibitors of the cognate cytokines.

Recently, other members of the TNFR family have been identified. Such newly identified members of the TNFR family include CAR1, HVEM and osteoprotegerin (OPG) [Brojatsch et al., Cell, 87:845-855 (1996); Montgomery et al., Cell, 87:427-436 (1996); Marsters et al., J. Biol. Chem., 272:14029-14032 (1997); Simonet et al., Cell, 89:309-319 (1997)]. Unlike other known TNFR-like molecules, Simonet et al., supra, report that OPG contains no hydrophobic transmembrane-spanning sequence.

In Masters et al., Curr. Biol., 6:750 (1996), investigators describe a full length native sequence human polypeptide, called Apo-3, which exhibits similarity to the TNFR family in its extracellular cysteine-rich repeats and resembles TNFR1 and CD95 in that it contains a cytoplasmic death domain sequence [see also Marsters et al., Curr. Biol., 6:1669 (1996)]. Apo-3 has also been referred to by other investigators as DR3, wsl-1 and TRAMP [Chinnaiyan et al., Science, 274:990 (1996); Kitson et al., Nature, 384:372 (1996); Bodmer et al., Immunity, 6:79 (1997)].

Pan et al. have disclosed another TNF receptor family member referred to as "DR4" [Pan et al., Science, 276:111-113 (1997)]. The DR4 was reported to contain a cytoplasmic death domain capable of engaging the cell suicide apparatus. Pan et al. disclose that DR4 is believed to be a receptor for the ligand known as Apo-2 ligand or TRAIL.

In Sheridan et al., Science, 277:818-821 (1997) and Pan et al., Science, 277:815-818 (1997), another molecule believed to be a receptor for the Apo-2 ligand (TRAIL) is described. That molecule is referred to as DR5 (it has also been alternatively referred to as Apo-2). Like DR4, DR5 is reported to contain a cytoplasmic death domain and be capable of signaling apoptosis.

In Sheridan et al., supra, a receptor called DcR1 (or alternatively, Apo-2DcR) is disclosed as being a potential decoy receptor for Apo-2 ligand (TRAIL). Sheridan et al. report that DcR1 can inhibit Apo-2 ligand function *in vitro.* See also, Pan et al., supra, for disclosure on the decoy receptor referred to as TRID.

For a review of the TNF family of cytokines and their receptors, see Gruss and Dower, supra.

As presently understood, the cell death program contains at least three important elements - activators, inhibitors, and effectors; in *C. elegans,* these elements are encoded respectively by three genes, *Ced-4, Ced-9* and Ced-3 [Steller, Science, 267:1445 (1995); Chinnaiyan et al., Science, 275:1122-1126 (1997); Wang et al., Cell, 90:1-20 (1997)]. Two of the TNFR family members, TNFR1 and Fas/Apo1 (CD95), can activate apoptotic cell death [Chinnaiyan and Dixit, Current Biology, 6:555-562 (1996); Fraser and Evan, Cell; 85:781-784 (1996)]. TNFR1 is also known to mediate activation of the transcription factor, NF-κB [Tartaglia et al., Cell, 74:845-853 (1993); Hsu et al., Cell, 84:299-308 (1996)]. In addition to some ECD homology, these two receptors share homology in their intracellular domain (ICD) in an oligomerization interface known as the death domain [Tartaglia et al., supra; Nagata, Cell, 88:355 (1997)]. Death domains are also found in several metazoan proteins that regulate apoptosis, namely, the Drosophila protein, Reaper, and the mammalian proteins referred to as FADD/MORT1, TRADD, and RIP [Cleaveland and Ihle, Cell, 81:479-482 (1995)]. Upon ligand binding and receptor clustering, TNFR1 and CD95 are believed to recruit FADD into a death-inducing signalling complex. CD95 purportedly binds FADD directly, while TNFR1 binds FADD indirectly via TRADD [Chinnaiyan et al., Cell, 81:505-512 (1995); Boldin et al., J. Biol. Chem., 270:387-391 (1995); Hsu et al., supra; Chinnaiyan et al., J. Biol. Chem., 271:4961-4965 (1996)]. It has been reported that FADD serves as an adaptor protein which recruits the Ced-3-related protease, MACHα/FLICE (caspase 8), into the death signalling complex [Boldin et al., Cell, 85:803-815 (1996); Muzio et al., Cell, 85:817-827 (1996)]. MACHα/FLICE appears to be the trigger that sets off a cascade of apoptotic proteases, including the interleukin-1β converting enzyme (ICE) and CPP32/Yama, which may execute some critical aspects of the cell death programme [Fraser and Evan, supra].

It was recently disclosed that programmed cell death involves the activity of members of a family of cysteine proteases related to the *C. elegans* cell death gene, *ced-3*, and to the mammalian IL-1-converting enzyme, ICE. The activity of the ICE and CPP32/Yama proteases can be inhibited by the product of the cowpox virus gene, crmA [Ray et al., Cell, 69:597-604 (1992); Tewari et al., Cell, 81:801-809 (1995)]. Recent studies show that CrmA can inhibit TNFR1- and CD95-induced cell death [Enari et al., Nature, 375:78-81 (1995); Tewari et al., J. Biol. Chem., 270:3255-3260 (1995)].

As reviewed recently by Tewari et al., TNFR1, TNFR2 and CD40 modulate the expression of proinflammatory and costimulatory cytokines, cytokine receptors, and cell adhesion molecules through activation of the transcription factor, NF-κB (Tewari et al., Curr. Op. Genet. Develop., 6:39-44 (1996)]. NF-κB is the prototype of a family of dimeric transcription factors whose subunits contain conserved Rel regions [Verma et al., Genes Develop., 9:2723-2735 (1996); Baldwin, Ann. Rev. Immunol., 14:649-681 (1996)]. In its latent form, NF-κB is complexed with members of the IκB inhibitor family; upon inactivation of the IκB in response to certain stimuli, released NF-κB translocates to the nucleus where it binds to specific DNA sequences and activates gene transcription. NF-κB is induced by a variety of proinflammatory signals and cytokines including IL-1 and LPS. acting through the Toll-like receptor TLR2 [Baeuerle et al., Ann. Rev. Immunol., 12:141-79 (1994); Verma et al., supra].

### SUMMARY OF THE INVENTION

Applicants have identified a cDNA clone that encodes a novel polypeptide, designated in the present application as "DNA19355. WO98/07880 refers to a polynucleotide sequence and its putative amino acid sequence (that corresponds to amino acids 9 to 177 of DNA 19355 polypeptide).

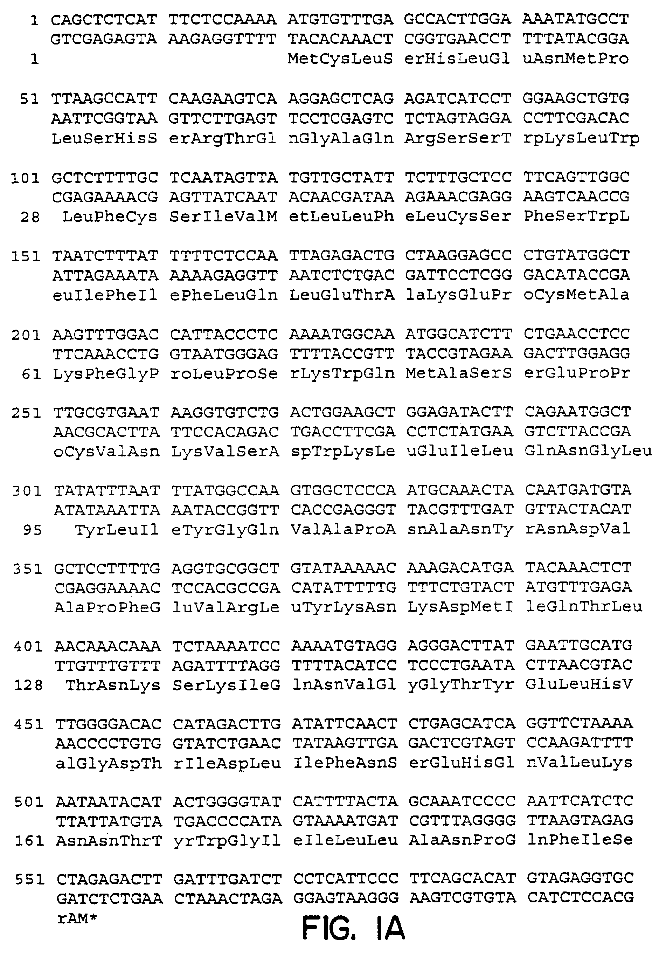

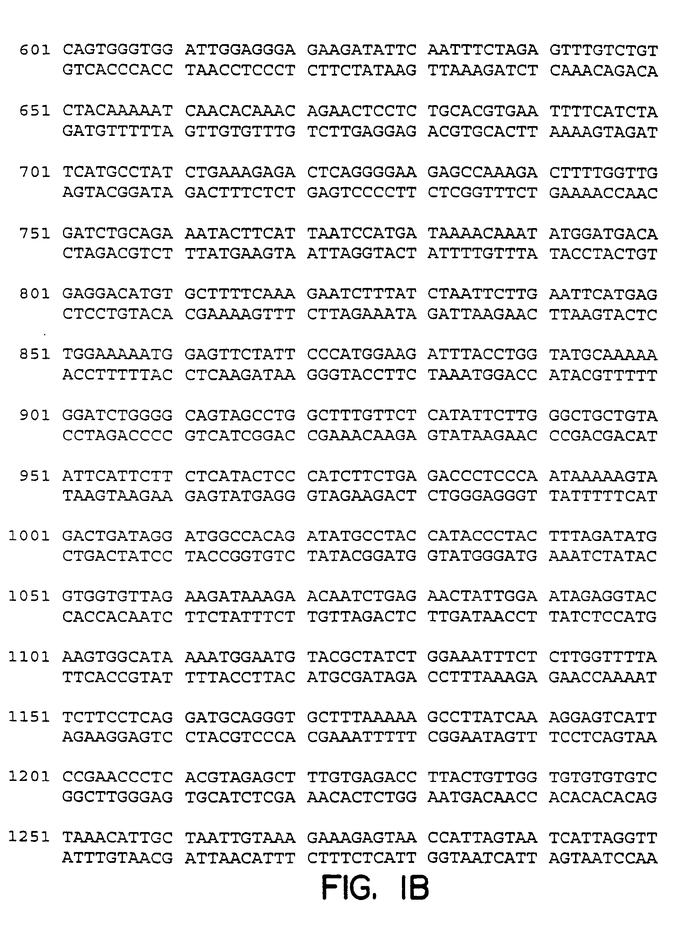

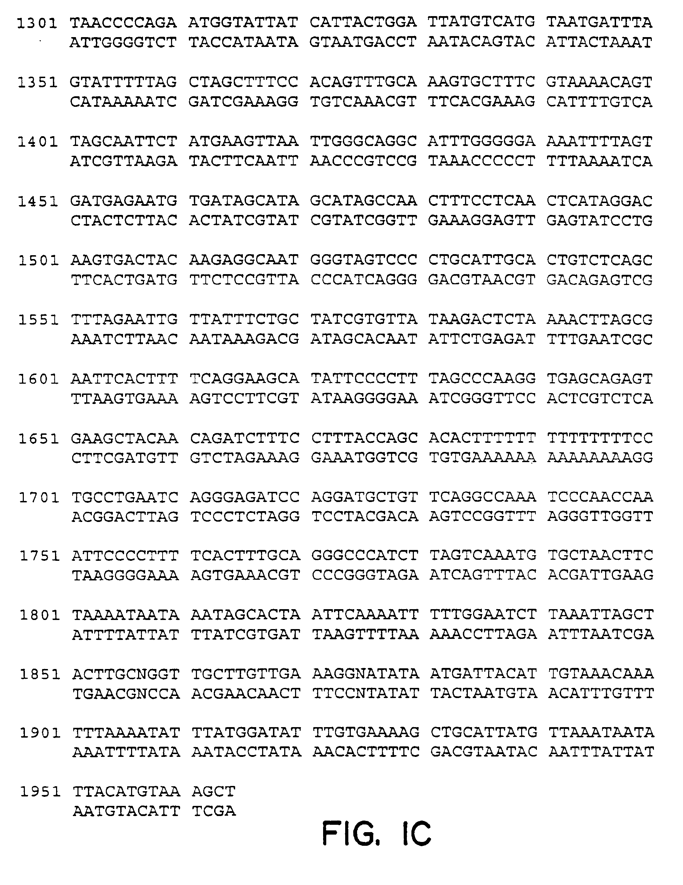

In one embodiment, the invention provides an isolated nucleic acid molecule comprising DNA encoding DNA19355 polypeptide as defined in the claims. Optionally, the isolated nucleic acid comprises DNA encoding DNA19355 polypeptide having amino acid residues 1 to 177 of Fig. 1 (SEQ ID NO:1), or is complementary to such encoding nucleic acid sequence, and remains stably bound to it under at least moderate, and optionally, under high stringency conditions. The isolated nucleic acid may comprise the DNA19355 cDNA insert of the vector deposited as ATCC 209466, and particular the insert which includes the DNA sequence encoding DNA19355 polypeptide.

In another embodiment, the invention provides a vector comprising DNA encoding DNA19355 polypeptide as defined in the claims. A host cell comprising such a vector is also provided. By way of example, the host cells may be CHO cells, *E. coli,* or yeast. A process for producing DNA19355 polypeptides is further provided and comprises culturing host cells under conditions suitable for expression of DNA19355 and recovering DNA19355 from the cell culture.

In another embodiment, the invention provides isolated DNA19355 polypeptide as defined in the claims. In particular, the invention provides isolated native sequence DNA19355 polypeptide, which in one embodiment, includes an amino acid sequence comprising residues 1 to 177 of Figure 1 (SEQ ID NO:1). Optionally, the DNA19355 polypeptide is obtained or obtainable by expressing the polypeptide encoded by the cDNA insert of the vector deposited as ATCC 209466.

Described herein are isolated DNA19355 polypeptide variants. The variants comprise polypeptides which have at least about 80% amino acid sequence identity with the deduced amino acid sequence of Fig. 1 (SEQ ID NO:1) or domain sequences identified herein, and preferably have activity(s) of native sequence or naturally-occurring DNA19355 polypeptide.

In another embodiment, the invention provides chimeric molecules comprising DNA19355 polypeptide fused to a heterologous polypeptide or amino acid sequence as defined in the claims. An example of such a chimeric molecule comprises a DNA19355 fused to an epitope tag sequence or a Fc region of an immunoglobulin.

Also described herein is an antibody which specifically binds to DNA19355 polypeptide. Optionally, the antibody is a monoclonal antibody. Also described herein are diagnostic and therapeutic methods using DNA19355. For example, methods of inducing apoptosis in mammalian cancer cells are described.

### BRIEF DESCRIPTION OF THE DRAWINGS

Figure 1 shows the nucleotide sequence (SEQ ID NO:2) of a cDNA for human DNA19355 and its derived amino acid sequence (SEQ ID NO:1).
Figure 2 shows an alignment and comparison of extracellular amino acid sequence of DNA19355 polypeptide with human Apo-2L, Fas/Apo1 ligand (CD95L), TNF-alpha and LT-α; the respective amino acid identities (%) are approximately 19.8, 19.0, 20.6, and 17.5.
Figure 3 shows a Northern blot analysis of DNA19355 mRNA expression in human tissues (identified adult and fetal tissues) and tumor cell lines (HL60 promyelocytic leukemia, HeLa S3 cervical carcinoma, K562 chronic myelogenous leukemia, MOLT4 lymphoblastic leukemia, Raji Burkitt's lymphoma, SW480 colorectal adenocarcinoma, A549 lung carcinoma, and G361 melanoma).
Figure 4 shows an analysis of soluble DNA19355 polypeptide by SDS-PAGE.
Figure 5 shows: (A) fluorescence images of Hoechst-stained nuclei from cells transfected with pRK5 (a); pRK5 encoding DNA19355 (b); pRK5 encoding Apo-2 ligand (c); pRK5 encoding DNA19355 plus pRK5 encoding CrmA (d); pRK5 encoding DNA19355 plus pRK5 encoding FADD-DN (e); pRK5 encoding Apo-2 ligand plus pRK5 encoding FADD-DN (f). (B) induction of apoptosis by transfected DNA19355 or Apo-2 ligand and effect of caspase inhibitors and FADD-DN.
Figure 6 shows the effect of DNA19355 on NF-κB activity. Electrophoretic mobility shift analysis of NF-κB activity in cells transfected with pRK5, or pRK5 encoding DNA19355, or pRK5 encoding Apo-2 ligand. In each case, the cells were co-transfected with pRK5 (left 3 lanes) or pRK5 encoding dominant-negative NIK (NIK-DN, right 3 lanes).
Figure 7 shows an alignment and comparison of the amino acid sequences for human GITR (hGITR) and murine GITR (mGITR). The three cysteine rich domains (CRD1, CRD2, and CRD3) and transmembrane region (TM) are shown.
Figure 8 shows the results of a co-precipitation assay described in Example 12 below. The autoradiograph of the SDS-PAGE gel revealed the hGITR-IgG molecule bound to the radioiodinated DNA19355 polypeptide. Binding was not observed for the other immunoadhesin constructs identified.
Figure 9A shows the results of FACS analysis of transfected 293 cells assayed for binding to the identified receptors or ligand immunoadhesin constructs.
Figure 9B shows the results of FACS analysis of HUVEC cells assayed for binding to the identified receptor immunoadhesin constructs.
Figure 10 shows the results of a luciferase activity assay conducted to demonstrate NF-κB activation by DNA19355/hGITR.
Figure 11 shows the results of an ELISA conducted to determine TNF-alpha levels in culture supernatants from primary T cells and monocytes/macrophages incubated with DNA19355 polypeptide.

### DETAILED DESCRIPTION OF THE PREFERRED EMBODIMENTS

### I. Definitions

The terms "DNA19355 polypeptide" and "DNA19355" when used herein encompass native sequence DNA19355 and DNA19355 variants (which are further defined herein). The DNA19355 may be isolated from a variety of sources, such as from human tissue types or from another source, or prepared by recombinant or synthetic methods. The terms "DNA19355 polypeptide" and "DNA19355" when used herein refer to the same polypeptides referred to in the literature as "GLITTER". A "native sequence DNA19355" comprises a polypeptide having the same amino acid sequence as an DNA19355 derived from nature. Such native sequence DNA19355 can be isolated from nature or can be produced by recombinant or synthetic means. The term "native sequence DNA19355" specifically encompasses naturally-occurring truncated, soluble or secreted forms of the DNA19355 (*e.g.*, an extracellular domain sequence or soluble form), naturally-occurring variant forms (*e.g.,* alternatively spliced forms) and naturally-occurring allelic variants of the DNA19355. In one embodiment of the invention, the native sequence DNA19355 is a mature or full-length native sequence DNA19355 polypeptide comprising amino acids 1 to 177 of Fig. 1 (SEQ ID NO:1). Alternatively, the DNA19355 polypeptide comprises amino acids 52 to 177 of Fig. 1 (SEQ ID NO:1). Optionally, the DNA19355 polypeptide is obtained or obtainable by expressing the polypeptide encoded by the cDNA insert of the vector deposited as ATCC 209466.

The "DNA19355 extracellular domain" or "DNA19355 ECD" refers to a form of DNA19355 which is essentially free of the transmembrane and cytoplasmic domains of DNA19355. Ordinarily, DNA19355 ECD will have less than 1% of such transmembrane and/or cytoplasmic domains and preferably, will have less than 0.5% of such domains. Optionally, DNA19355 ECD will comprise amino acid residues X to 177 of Fig. 1 (SEQ ID NO:1), wherein X is any one of amino acid residues 48 to 57 of Fig. 1 (SEQ ID NO:1). It will be understood by the skilled artisan that the transmembrane domain identified for the DNA19355 polypeptide of the present invention is identified pursuant to criteria routinely employed in the art for identifying that type of hydrophobic domain. The exact boundaries of a transmembrane domain may vary but most likely by no more than about 5 amino acids at either end of the domain specifically mentioned herein.

"DNA19355 variant" means a DNA19355 as defined below having at least about 80% amino acid sequence identity with the DNA19355 having the deduced amino acid sequence shown in Fig. 1 (SEQ ID NO:1) for a full-length native sequence DNA19355 or the various domain sequences identified herein. Such DNA19355 variants include, for instance, DNA19355 polypeptides wherein one or more amino acid residues are added, or deleted, at the N- or C-terminus of the sequence of Fig. 1 (SEQ ID NO:1). DNA19355 variants include ECD fragments which include sequences having less than amino acid residues 52 to 177 of Fig. 1 (SEQ ID NO:1). Ordinarily, a DNA19355 variant will have at least about 80% or 85% amino acid sequence identity, more preferably at least about 90% amino acid sequence identity, and even more preferably at least about 95% amino acid sequence identity with the amino acid sequence of Fig. 1 (SEQ ID NO:1).

"Percent (%) amino acid sequence identity" with respect to the DNA19355 sequences identified herein is defined as the percentage of amino acid residues in a candidate sequence that are identical with the amino acid residues in the DNA19355 sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, ALIGN or Megalign (DNASTAR) software. Those skilled in the art can determine appropriate parameters for measuring alignment, including any algorithms needed to achieve maximal alignment over the full length of the sequences being compared.

"Percent (%) nucleic acid sequence identity" with respect to the DNA19355 sequences identified herein is defined as the percentage of nucleotides in a candidate sequence that are identical with the nucleotides in the DNA19355 sequence, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, ALIGN or Megalign (DNASTAR) software. Those skilled in the art can determine appropriate parameters for measuring alignment, including any algorithms needed to achieve maximal alignment over the full length of the sequences being compared.

The term "epitope tagged" when used herein refers to a chimeric polypeptide comprising DNA19355, or a domain sequence thereof, fused to a "tag polypeptide". The tag polypeptide has enough residues to provide an epitope against which an antibody can be made, or which can be identified by some other agent, yet is short enough such that it does not interfere with activity of the DNA19355. The tag polypeptide preferably also is fairly unique so that the antibody does not substantially cross-react with other epitopes. Suitable tag polypeptides generally have at least six amino acid residues and usually between about 8 to about 50 amino acid residues (preferably, between about 10 to about 20 residues).

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide in situ within recombinant cells, since at least one component of the DNA19355 natural environment will not be present. Ordinarily, however, isolated polypeptide will be prepared by at least one purification step.

An "isolated" DNA19355 nucleic acid molecule is a nucleic acid molecule that is identified and separated from at least one contaminant nucleic acid molecule with which it is ordinarily associated in the natural source of the DNA19355 nucleic acid. An isolated DNA19355 nucleic acid molecule is other than in the form or setting in which it is found in nature. Isolated DNA19355 nucleic acid molecules therefore are distinguished from the DNA19355 nucleic acid molecule as it exists in natural cells. However, an isolated DNA19355 nucleic acid molecule includes DNA19355 nucleic acid molecules contained in cells that ordinarily express DNA19355 where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadest sense and specifically covers single anti-DNA19355 monoclonal antibodies (including agonist, antagonist, and neutralizing antibodies) and anti-DNA19355 antibody compositions with polyepitopic specificity. The term "monoclonal antibody" as used herein refers to an antibody obtained from a population of substantially homogeneous antibodies, i.e., the individual antibodies comprising the population are identical except for possible naturally-occurring mutations that may be present in minor amounts.

"Biologically active" and "desired biological activity" for the purposes herein mean (1) having the ability to modulate apoptosis (either in an agonistic or stimulating manner or in an antagonistic or blocking manner) in at least one type of mammalian cell *in vivo* or *ex vivo* or (2) having the ability to induce or stimulate a proinflammatory response in at least one type of mammalian cell *in vivo* or *ex vivo.*

The terms "apoptosis" and "apoptotic activity" are used in a broad sense and refer to the orderly or controlled form of cell death in mammals that is typically accompanied by one or more characteristic cell changes, including condensation of cytoplasm, loss of plasma membrane microvilli, segmentation of the nucleus, degradation of chromosomal DNA or loss of mitochondrial function. This activity can be determined and measured, for instance, by cell viability assays, FACS analysis or DNA electrophoresis, all of which are known in the art.

The terms "treating," "treatment," and "therapy" as used herein refer to curative therapy, prophylactic therapy, and preventative therapy.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers include squamous cell cancer, small-cell lung cancer, non-small-cell lung cancer, blastoma, gastrointestinal cancer, renal cancer, pancreatic cancer, glioblastoma, neuroblastoma, cervical cancer, ovarian cancer, liver cancer, stomach cancer, bladder cancer, hepatoma, breast cancer, colon cancer, colorectal cancer, endometrial cancer, salivary gland cancer, kidney cancer, prostate cancer, vulval cancer, thyroid cancer, hepatic carcinoma, and various types of head and neck cancer.

The term "mammal" as used herein refers to any mammal classified as a mammal, including humans, cows, horses, dogs and cats. In a preferred embodiment of the invention, the mammal is a human.

### II. Compositions and Methods of the Invention

### A. DNA19355 Polypeptides

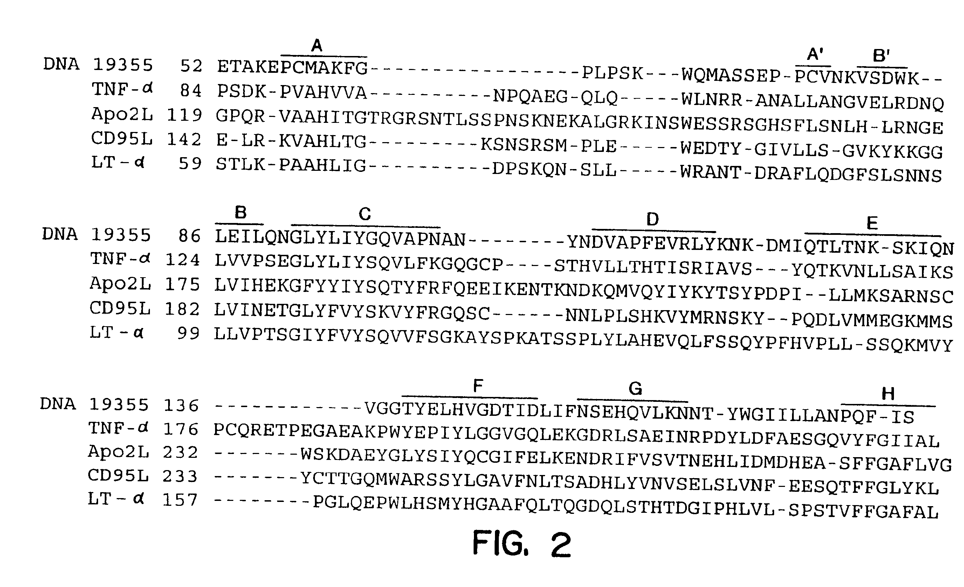

The present invention provides newly identified and isolated nucleotide sequences encoding polypeptides referred to in the present application as DNA19355. In particular, Applicants have identified and isolated cDNA encoding a DNA19355 polypeptide, as disclosed in further detail in the Examples below. Using BLAST and FastA sequence alignment computer programs, Applicants found that DNA19355 (shown in Figure 1 and SEQ ID NO:1) shares certain amino acid sequence identity with some members of the TNF family (see, e.g., Figure 2 and Example 1 below). As shown in the Examples below, DNA19355 polypeptide was found to have apoptotic activity and specific binding to GITR. It was also found that DNA19355 stimulated secretion of TNF-alpha in primary T cells *in vitro* (Example 14) and infiltrate or influx of neutrophils in a guinea pig skin biopsy assay (such as described in Example 15), suggesting the role of DNA19355 in proinflammatory responses.

In addition to the full-length native sequence DNA19355 and soluble forms of DNA19355 described herein, it is contemplated that DNA19355 variants can be prepared. DNA19355 variants can be prepared by introducing appropriate nucleotide changes into the DNA19355 nucleotide sequence, or by synthesis of the desired DNA19355 polypeptide. Those skilled in the art will appreciate that amino acid changes may alter post-translational processes of the DNA19355, such as changing the number or position of glycosylation sites or altering the membrane anchoring characteristics.

Variations in the native full-length sequence DNA19355 or in various domains of the DNA19355 described herein, can be made, for example, using any of the techniques and guidelines for conservative and non-conservative mutations set forth, for instance, in U.S. Patent No. 5,364,934. Variations may be a substitution, deletion or insertion of one or more codons encoding the DNA19355 that results in a change in the amino acid sequence of the DNA19355 as compared with the native sequence DNA19355. Optionally the variation is by substitution of at least one amino acid with any other amino acid in one or more of the domains of the DNA19355. Guidance in determining which amino acid residue may be inserted, substituted or deleted without adversely affecting the desired activity may be found by comparing the sequence of the DNA19355 with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of 1 to 5 amino acids. The variation allowed may be determined by systematically making insertions, deletions or substitutions of amino acids in the sequence and testing the resulting variants for activity in any of the *in vitro* assays described in the Examples below.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6487 (1987)], cassette mutagenesis [wells et al., Gene, 34:315 (1985)], restriction selection mutagenesis [Wells et al., Philos. Trans. R. Soc. London SerA, 317:415 (1986)] or other known techniques can be performed on the cloned DNA to produce the DNA19355 variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

### B. Modifications of DNA19355

Covalent modifications of DNA19355 are included within the scope of this invention. One type of covalent modification includes reacting targeted amino acid residues of the DNA19355 with an organic derivatizing agent that is capable of reacting with selected side chains or the N- or C- terminal residues of the DNA19355. Derivatization with bifunctional agents is useful, for instance, for crosslinking DNA19355 to a water-insoluble support matrix or surface for use in the method for purifying anti-DNA19355 antibodies, and vice-versa. Commonly used crosslinking agents include, *e.g.*, 1,1-bis(diazoacetyl)-2-phenylethane, glutaraldehyde, N-hydroxysuccinimide esters, for example, esters with 4-azido-salicylic acid, homobifunctional imidoesters, including disuccinimidyl esters such as 3,3'-dithiobis(succinimidyl-propionate), bifunctional maleimides such as bis-N-maleimido-1,8-octane and agents such as methyl-3-[(p-azidophenyl)dithio]propioimi-date.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the α-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the DNA19355 polypeptide included within the scope of this invention comprises altering the native glycosylation pattern of the polypeptide. "Altering the native glycosylation pattern" is intended for purposes herein to mean deleting one or more carbohydrate moieties found in native sequence DNA19355, and/or adding one or more glycosylation sites that are not present in the native sequence DNA19355.

Addition of glycosylation sites to the DNA19355 polypeptide may be accomplished by altering the amino acid sequence. The alteration may be made, for example, by the addition of, or substitution by, one or more serine or threonine residues to the native sequence DNA19355 (for O-linked glycosylation sites). The DNA19355 amino acid sequence may optionally be altered through changes at the DNA level, particularly by mutating the DNA encoding the DNA19355 polypeptide at preselected bases such that codons are generated that will translate into the desired amino acids.

Another means of increasing the number of carbohydrate moieties on the DNA19355 polypeptide is by chemical or enzymatic coupling of glycosides to the polypeptide. Such methods are described in the art, e.g., in WO 87/05330 published 11 September 1987, and in Aplin and Wriston, CRC Crit. Rev. Biochem., pp. 259-306 (1981).

Removal of carbohydrate moieties present on the DNA19355 polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of DNA19355 comprises linking the DNA19355 polypeptide to one of a variety of nonproteinaceous polymers, *e.g.*, polyethylene glycol, polypropylene glycol, or polyoxyalkylenes, in the manner set forth in U.S. Patent Nos. 4,640,835; 4,496,689; 4,301,144; 4,670,417; 4,791,192 or 4,179,337.

The DNA19355 of the present invention may also be modified in a way to form a chimeric molecule comprising DNA19355 fused to another, heterologous polypeptide or amino acid sequence. In one embodiment, such a chimeric molecule comprises a fusion of the DNA19355 with a tag polypeptide which provides an epitope to which an anti-tag antibody can selectively bind. The epitope tag is generally placed at the amino- or carboxyl- terminus of the DNA19355. The presence of such epitope-tagged forms of the DNA19355 can be detected using an antibody against the tag polypeptide. Also, provision of the epitope tag enables the DNA19355 to be readily purified by affinity purification using an anti-tag antibody or another type of affinity matrix that binds to the epitope tag. In an alternative embodiment, the chimeric molecule may comprise a fusion of the DNA19355 with an immunoglobulin or a particular region of an immunoglobulin. For a bivalent form of the chimeric molecule, such a fusion could be to the Fc region of an IgG molecule. In particular, the chimeric molecule may comprise a DNA19355 ECD fused to a His-tag molecule.

Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an α-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

The DNA19355 of the invention may also be modified in a way to form a chimeric molecule comprising DNA19355 fused to a leucine zipper. Various leucine zipper polypeptides have been described in the art. See, e.g., Landschulz et al., Science, 240:1759 (1988); WO 94/10308; Hoppe et al., FEBS Letters, 344:1991 (1994); Maniatis et al., Nature, 341:24 (1989). It is believed that use of a leucine zipper fused to DNA19355 may be desirable to assist in dimerizing or trimerizing soluble DNA19355 in solution. Those skilled in the art will appreciate that the leucine zipper may be fused at either the 5' or 3' end of the DNA19355 molecule.

### C. Preparation of DNA19355

The description below relates primarily to production of DNA19355 by culturing cells transformed or transfected with a vector containing DNA19355 nucleic acid. It is, of course, contemplated that alternative methods, which are well known in the art, may be employed to prepare DNA19355. For instance, the DNA19355 sequence, or portions thereof, may be produced by direct peptide synthesis using solid-phase techniques [see, e.g., Stewart et al., Solid-Phase Peptide Synthesis, W.H. Freeman Co., San Francisco, CA (1969); Merrifield, J. Am. Chem. Soc., 85:2149-2154 (1963)]. In vitro protein synthesis may be performed using manual techniques or by automation. Automated synthesis may be accomplished, for instance, using an Applied Biosystems Peptide Synthesizer (Foster City, CA) using manufacturer's instructions. Various portions of the DNA19355 may be chemically synthesized separately and combined using chemical or enzymatic methods to produce the full-length DNA19355.

### 1. Isolation of DNA Encoding DNA19355

DNA encoding DNA19355 may be obtained from a cDNA library prepared from tissue believed to possess the DNA19355 mRNA and to express it at a detectable level. Accordingly, human DNA19355 DNA can be conveniently obtained from a cDNA library prepared from human tissue. The DNA19355-encoding gene may also be obtained from a genomic library or by oligonucleotide synthesis.

Libraries can be screened with probes (such as antibodies to the DNA19355 or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding DNA19355 is to use PCR methodology [Sambrook et al., supra; Dieffenbach et al., PCR Primer:A Laboratory Manual (Cold Spring Harbor Laboratory Press, 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like ³²P-labeled ATP, biotinylation or enzyme labeling. Hybridization conditions, including moderate stringency and high stringency, are provided in Sambrook et al., supra.

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined through sequence alignment using computer software programs such as ALIGN, DNAstar, and INHERIT which employ various algorithms to measure homology.

Nucleic acid having protein coding sequence may be obtained by screening selected cDNA or genomic libraries using the deduced amino acid sequence disclosed herein for the first time, and, if necessary, using conventional primer extension procedures as described in Sambrook et al., supra, to detect precursors and processing intermediates of mRNA that may not have been reverse-transcribed into cDNA.

### 2. Selection and Transformation of Host Cells

Host cells are transfected or transformed with expression or cloning vectors described herein for DNA19355 production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., supra.

Methods of transfection are known to the ordinarily skilled artisan, for example, CaPO₄ and electroporation. Depending on the host cell used, transformation is performed using standard techniques appropriate to such cells. The calcium treatment employing calcium chloride, as described in Sambrook et al., supra, or electroporation is generally used for prokaryotes or other cells that contain substantial cell-wall barriers. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transformations have been described in U.S. Patent No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, *e.g.*, polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E. coli* strains are publicly available, such as *E. coli* K12 strain MM294 (ATCC 31,446); *E. coli* X1776 (ATCC 31,537); *E. coli* strain W3110 (ATCC 27,325) and K5 772 (ATCC 53,635).

In addition to prokaryotes, eukaryotic microbes such as filamentous fungi or yeast are suitable cloning or expression hosts for DNA19355-encoding vectors. *Saccharomyces cerevisiae* is a commonly used lower eukaryotic host microorganism.

Suitable host cells for the expression of glycosylated DNA19355 are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV1 line transformed by SV40 (COS-7, ATCC CRL 1651); human embryonic kidney line (293 or 293 cells subcloned for growth in suspension culture, Graham et al., J. Gen Virol., 36:59 (1977)); Chinese hamster ovary cells/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); human lung cells (W138, ATCC CCL 75); human liver cells (Hep G2, HB 8065); and mouse mammary tumor (MMT 060562, ATCC CCL51). The selection of the appropriate host cell is deemed to be within the skill in the art.

### 3. Selection and Use of a Replicable Vector

The nucleic acid (*e.g.*, cDNA or genomic DNA) encoding DNA19355 may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The DNA19355 may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the DNA19355 DNA that is inserted into the vector. The signal sequence may be a prokaryotic signal sequence selected, for example, from the group of the alkaline phosphatase, penicillinase, lpp, or heat-stable enterotoxin II leaders. For yeast secretion the signal sequence may be, *e.g.*, the yeast invertase leader, alpha factor leader (including *Saccharomyces* and *Kluyveromyces* α-factor leaders, the latter described in U.S. Patent No. 5,010,182), or acid phosphatase leader, the *C. albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, *e.g.*, ampicillin, neomycin, methotrexate, or tetracycline, (b) complement auxotrophic deficiencies, or (c) supply critical nutrients not available from complex media, *e.g.*, the gene encoding D-alanine racemase for *Bacilli*.

An example of suitable selectable markers for mammalian cells are those that enable the identification of cells competent to take up the DNA19355 nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10:157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the DNA19355 nucleic acid sequence to direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding DNA19355.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

DNA19355 transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, *e.g.*, the actin promoter or an immunoglobulin promoter, and from heat-shock promoters, provided such promoters are compatible with the host cell systems.

Transcription of a DNA encoding the DNA19355 by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the DNA19355 coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding DNA19355.

Still other methods, vectors, and host cells suitable for adaptation to the synthesis of DNA19355 in recombinant vertebrate cell culture are described in Gething et al., Nature, 293:620-625 (1981); Mantei et al., Nature, 281:40-46 (1979); EP 117,060; and EP 117,058.

### 4. Detecting Gene Amplification/Expression

Gene amplification and/or expression may be measured in a sample directly, for example, by conventional Southern blotting, Northern blotting to quantitate the transcription of mRNA [Thomas, Proc. Natl. Acad. Sci. USA, 77:5201-5205 (1980)], dot blotting (DNA analysis), or in situ hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence DNA19355 polypeptide or against a synthetic peptide based on the DNA sequences provided herein or against exogenous sequence fused to DNA19355 DNA and encoding a specific antibody epitope.

### 5. Purification of Polypeptide

Forms of DNA19355 may be recovered from culture medium or from host cell lysates. If membrane-bound, it can be released from the membrane using a suitable detergent solution (*e.g.* Triton-X 100) or by enzymatic cleavage. Cells employed in expression of DNA19355 can be disrupted by various physical or chemical means, such as freeze-thaw cycling, sonication, mechanical disruption, or cell lysing agents.

It may be desired to purify DNA19355 from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the DNA19355. Various methods of protein purification may be employed and such methods are known in the art and described for example in Deutscher, Methods in Enzymology, 182 (1990); Scopes, Protein Purification:Principles and Practice, Springer-verlag, New York (1982). The purification step(s) selected will depend, for example, on the nature of the production process used and the particular DNA19355 produced.

### D. Uses for DNA19355

Nucleotide sequences (or their complement) encoding DNA19355 have various applications in the art of molecular biology, including uses as hybridization probes, in chromosome and gene mapping and in the generation of anti-sense RNA and DNA. DNA19355 nucleic acid will also be useful for the preparation of DNA19355 polypeptides by the recombinant techniques described herein.

The full-length native sequence DNA19355 (Fig. 1; SEQ ID NO:2) gene, or portions thereof, may be used as hybridization probes for a cDNA library to isolate, for instance, still other genes (like those encoding naturally-occurring variants of DNA19355 or DNA19355 from other species) which have a desired sequence identity to the DNA19355 sequence disclosed in Fig. 1 (SEQ ID NO:2). Optionally, the length of the probes will be about 20 to about 50 bases. The hybridization probes may be derived from the nucleotide sequence of SEQ ID NO:2 or from genomic sequences including promoters, enhancer elements and introns of native sequence DNA19355. By way of example, a screening method will comprise isolating the coding region of the DNA19355 gene using the known DNA sequence to synthesize a selected probe of about 40 bases. Hybridization probes may be labeled by a variety of labels, including radionucleotides such as ¹²P or ²⁵S, or enzymatic labels such as alkaline phosphatase coupled to the probe via avidin/biotin coupling systems. Labeled probes having a sequence complementary to that of the DNA19355 gene of the present invention can be used to screen libraries of human cDNA, genomic DNA or mRNA to determine which members of such libraries the probe hybridizes to. Hybridization techniques are described in further detail in the Examples below.

Nucleotide sequences encoding a DNA19355 can also be used to construct hybridization probes for mapping the gene which encodes that DNA19355 and for the genetic analysis of individuals with genetic disorders. The nucleotide sequences provided herein may be mapped to a chromosome and specific regions of a chromosome using known techniques, such as in situ hybridization, linkage analysis against known chromosomal markers, and hybridization screening with libraries.

Screening assays can be designed to find lead compounds that mimic the biological activity of a native sequence DNA19355 or a ligand or receptor for Data19355. Such screening assays will include assays amenable to high-throughput screening of chemical libraries, making them particularly suitable for identifying small molecule drug candidates. Small molecules contemplated include synthetic organic or inorganic compounds. The assays can be performed in a variety of formats, including protein-protein binding assays, biochemical screening assays, immunoassays and cell based assays, which are well characterized in the art.

Nucleic acids which encode DNA19355 or its modified forms can also be used to generate either transgenic animals or "knock out" animals which, in turn, are useful in the development and screening of therapeutically useful reagents. A transgenic animal (e.g., a mouse or rat) is an animal having cells that contain a transgene, which transgene was introduced into the animal or an ancestor of the animal at a prenatal, e.g., an embryonic stage. A transgene is a DNA which is integrated into the genome of a cell from which a transgenic animal develops. In one embodiment, cDNA encoding DNA19355 can be used to clone genomic DNA encoding DNA19355 in accordance with established techniques and the genomic sequences used to generate transgenic animals that contain cells which express DNA encoding DNA19355. Methods for generating transgenic animals, particularly animals such as mice or rats, have become conventional in the art and are described, for example, in U.S. Patent Nos. 4,736,866 and 4,870,009. Typically, particular cells would be targeted for DNA19355 transgene incorporation with tissue-specific enhancers. Transgenic animals that include a copy of a transgene encoding DNA19355 introduced into the germ line of the animal at an embryonic stage can be used to examine the effect of increased expression of DNA encoding DNA19355. Such animals can be used as tester animals for reagents thought to confer protection from, for example, pathological conditions associated with its overexpression. An animal can be treated with the reagent and a reduced incidence of the pathological condition, compared to untreated animals bearing the transgene, would indicate a potential therapeutic intervention for the pathological condition.

Alternatively, non-human homologues of DNA19355 can be used to construct a DNA19355 "knock out" animal which has a defective or altered gene encoding DNA19355 as a result of homologous recombination between the endogenous gene encoding DNA19355 and altered genomic DNA encoding DNA19355 introduced into an embryonic cell of the animal. For example, cDNA encoding DNA19355 can be used to clone genomic DNA encoding DNA19355 in accordance with established techniques. A portion of the genomic DNA encoding DNA19355 can be deleted or replaced with another gene, such as a gene encoding a selectable marker which can be used to monitor integration. Typically, several kilobases of unaltered flanking DNA (both at the 5' and 3' ends) are included in the vector [see e.g., Thomas and Capecchi, Cell, 51:503 (1987) for a description of homologous recombination vectors]. The vector is introduced into an embryonic stem cell line (e.g., by electroporation) and cells in which the introduced DNA has homologously recombined with the endogenous DNA are selected [see e.g., Li et al., Cell, 69:915 (1992)]. The selected cells are then injected into a blastocyst of an animal (e.g., a mouse or rat) to form aggregation chimeras [see e.g., Bradley, in Teratocarcinomas and Embryonic Stem Cells: A Practical Approach, E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the DNA19355 polypeptide.

The DNA19355 polypeptides may also be employed in diagnostic assays to, for instance, detect the presence of the receptor "GITR" in mammalian tissues. Such assays may be conducted using techniques known in the art or for example, using the binding assays described herein.

The DNA19355 polypeptides may further be employed as immunogens to raise antibodies against DNA19355. Techniques and methods for generating antibodies are described below.

The DNA19355 polypeptides can also be employed therapeutically. For example, the DNA19355 polypeptides can be employed to induce apoptosis in mammalian cancer cells. Generally, the methods for inducing apoptosis in mammalian cancer cells comprise exposing the cells to an effective (or apoptosis-inducing) amount of the DNA19355 polypeptide. Therapeutic application of DNA19355 polypeptide for the treatment of cancer is described in detail below.

In the methods for treating cancer, DNA19355 polypeptide is administered to a mammal diagnosed as having cancer. It is of course contemplated that the DNA19355 polypeptide can be employed in combination with still other therapeutic compositions and techniques, including other apoptosis-inducing agents, chemotherapy, radiation therapy and surgery.

The DNA19355 polypeptide is administered in an acceptable carrier, and preferably, a pharmaceutically-acceptable carrier. Suitable carriers and their formulations are described in Remington's Pharmaceutical Sciences, 16th Ed., 1980, Mack Publishing Co., edited by Oslo et al. Typically, an appropriate amount of a pharmaceutically-acceptable salt is used in the formulation to render the formulation isotonic. Examples of acceptable carriers include saline, Ringer's solution, and dextrose solution. The pH of the solution is preferably from about 5 to about 8, and more preferably, from about 7.4 to about 7.8. It will be apparent to those persons skilled in the art that certain carriers may be more preferable depending upon, for instance, the route of administration and concentration of the DNA19355 polypeptide being administered.

The DNA19355 polypeptide may be administered to a mammal by injection (e.g., intravenous, intraperitoneal, subcutaneous, intramuscular), or by other methods such as infusion that ensure its delivery to the bloodstream in an effective form. It is also contemplated that the DNA19355 polypeptide can be administered by in vivo or ex vivo gene therapy.

Effective dosages and schedules for administering DNA19355 polypeptide may be determined empirically, and making such determinations is within the skill in the art. It is presently believed that an effective dosage or amount of DNA19355 polypeptide may range from about 1 microgram/kg to about 100 mg/kg of body weight or more per day. Interspecies scaling of dosages can be performed in a manner known in the art, e.g., as disclosed in Mordenti et al., Pharmaceut. Res., 8:1351 (1991). Those skilled in the art will understand that the dosage of DNA19355 polypeptide that must be administered will vary depending on, for example, the mammal which will receive the DNA19355 polypeptide, the route of administration, and other drugs or therapies being administered to the mammal.

The one or more other therapies administered to the mammal may include but are not limited to, chemotherapy and/or radiation therapy, immunoadjuvants, cytokines, and antibody-based therapies. Examples include interleukins (e.g., IL-1, IL-2, IL-3, IL-6), leukemia inhibitory factor, interferons, erythropoietin, anti-VEGF antibody, and Her-2 antibody. Other agents known to induce apoptosis in mammalian cells may also be employed, and such agents include TNF-alpha, TNF-beta, CD30 ligand, 4-1BB ligand and Apo-1 ligand.

Chemotherapies include chemical substances or drugs which are known in the art and are commercially available, such as Doxorubicin, 5-Fluorouracil, etoposide, camptothecin, leucovorin, Cytosin arabinoside (Ara-C), Cyclophosphamide, Thiotepa, Busulfan, Cytoxin, Taxol, methotrexate, Cisplatin, Melphalin, Vinblastine, and Carboplatin. Preparation and dosing schedules for such chemotherapy may be used according to manufacturer's instructions or as determined empirically by the skilled practitioner. Preparation and dosing schedules for such chemotherapy are also described in Chemotherapy Service Ed., M.C. Perry, Williams & Wilkins, Baltimore, MD (1992).

The chemotherapy is administered in an acceptable carrier, preferably a pharmaceutically-acceptable carrier, such as those described above. The mode of administration of the chemotherapy may be the same as employed for the DNA19355 polypeptide or it may be administered to the mammal via a different mode. For example, the DNA19355 polypeptide may be injected while the chemotherapy is administered orally to the mammal.

Radiation therapy can be administered to the mammal according to protocols commonly employed in the art and known to the skilled artisan. Such therapy may include cesium, iridium, iodine or cobalt radiation. The radiation may be whole body irradiation, or may be directed locally to a specific site or tissue in or on the body. Typically, radiation therapy is administered in pulses over a period of time from about 1 to about 2 weeks. Optionally, the radiation therapy may be administered as a single dose or as multiple, sequential doses.

The DNA19355 polypeptide and one or more other therapies may be administered to the mammal concurrently or sequentially. Following administration of DNA19355 polypeptide and one or more other therapies to the mammal, the mammal's physiological condition can be monitored in various ways well known to the skilled practitioner. For instance, tumor mass may be observed physically, by biopsy, or by standard x-ray imaging techniques.

The modes and methods of administering DNA19355 polypeptide described above may also be used by the skilled practitioner to treat conditions whereby stimulation or induction of a proinflammatory response is desired.

### E. Anti-DNA19355 Antibodies

The present invention further describes herein anti-DNA19355 antibodies. Exemplary antibodies include polyclonal, monoclonal, humanized, bispecific, and heteroconjugate antibodies.

### 1. Polyclonal Antibodies

The DNA19355 antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the DNA19355 polypeptide or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

### 2. Monoclonal Antibodies

The DNA19355 antibodies may, alternatively, be monoclonal antibodies. Monoclonal antibodies may be prepared using hybridoma methods, such as those described by Kohler and Milstein, Nature, 256: 495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro*.

The immunizing agent will typically include the DNA19355 polypeptide or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against DNA19355. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

In vitro methods are also suitable for preparing monovalent antibodies. Digestion of antibodies to produce fragments thereof, particularly, Fab fragments, can be accomplished using routine techniques known in the art.

### 3. Humanized Antibodies

The DNA19355 antibodies may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321: 522-525 (1986); Riechmann et al., Nature, 332:323-329 (1988) ; and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321: 522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227 : 381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147 (1) :86-95 (1991)] .

### 4. Bispecific Antibodies

Bispecific antibodies are monoclonal, preferably human or humanized, antibodies that have binding specificities for at least two different antigens. In the present case, one of the binding specificities is for the DNA19355, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities [Milstein and Cuello, Nature, 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CHl) containing the site necessary for light-chain binding present in at least one of the fusions. DNAs encoding the immunoglobulin heavy-chain fusions and, if desired, the immunoglobulin light chain, are inserted into separate expression vector, and are co-transfected into a suitable host organism. For further details of generating bispecific antibodies see, for example, Suresh et al., Methods in Enzymology, 121:210 (1986).

### 5. Heteroconjugate Antibodies

Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared in vitro using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### F. Uses for DNA19355 Antibodies

The DNA19355 antibodies have various utilities. For example, DNA19355 antibodies may be used in diagnostic assays for DNA19355, *e.g.*, detecting its expression in specific cells, tissues, or serum. Various diagnostic assay techniques known in the art may be used, such as competitive binding assays, direct or indirect sandwich assays and immunoprecipitation assays conducted in either heterogeneous or homogeneous phases [Zola, Monoclonal Antibodies: A Manual of Techniques, CRC Press, Inc. (1987) pp. 147-158]. The antibodies used in the diagnostic assays can be labeled with a detectable moiety. The detectable moiety should be capable of producing, either directly or indirectly, a detectable signal. For example, the detectable moiety may be a radioisotope, such as ³H, ¹⁴C, ³²P, ³⁵S, or ¹²⁵I, a fluorescent or chemiluminescent compound, such as fluorescein isothiocyanate, rhodamine, or luciferin, or an enzyme, such as alkaline phosphatase, beta-galactosidase or horseradish peroxidase. Any method known in the art for conjugating the antibody to the detectable moiety may be employed, including those methods described by Hunter et al., Nature, 144:945 (1962); David et al., Biochemistry, 13:1014 (1974); Pain et al., J. Immunol. Meth., 40:219 (1981); and Nygren, J. Histochem and Cytochem., 30:407 (1982).

DNA19355 antibodies also are useful for the affinity purification of DNA19355 from recombinant cell culture or natural sources. In this process, the antibodies against DNA19355 are immobilized on a suitable support, such a Sephadex resin or filter paper, using methods well known in the art. The immobilized antibody then is contacted with a sample containing the DNA19355 to be purified, and thereafter the support is washed with a suitable solvent that will remove substantially all the material in the sample except the DNA19355, which is bound to the immobilized antibody. Finally, the support is washed with another suitable solvent that will release the DNA19355 from the antibody.

The following examples are offered for illustrative purposes only, and are not intended to limit the scope of the present invention in any way.

### EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, Virginia.

### EXAMPLE 1

### Isolation of Human DNA19355

Methods described in Klein et al., PNAS, 93:7108-7113 (1996) were employed with the following modifications. Yeast transformation was performed with limiting amounts of transforming DNA in order to reduce the number of multiple transformed yeast cells. Instead of plasmid isolation from the yeast followed by transformation of E. coli as described in Klein et al., supra, PCR analysis was performed on single yeast colonies. This was accomplished by restreaking the original sucrose positive colony onto fresh sucrose medium to purify the positive clone. A single purified colony was then used for PCR using the following primers: TGTAAAACGACGGCCAGTTTCTCTCAGAGAAACAAGCAAAAC (SEQ ID NO : 7) and CAGGAAACAGCTATGACCGAAGTGGACCAAAGGTCTATCGCTA (SEQ ID NO : 8). The PCR primers are bipartite in order to amplify the insert and a small portion of the invertase gene (allowing to determine that the insert was in frame with invertase) and to add on universal sequencing primer sites.

A library of cDNA fragments derived from human HUVEC cells fused to invertase was transformed into yeast and transformants were selected on SC-URA media. URA and transformants were replica plated onto sucrose medium in order to identify clones secreting invertase. Positive clones were re-tested and PCR products were sequenced. The sequence of one clone, DNA1840, was determined to contain a signal peptide coding sequence. Oligonucleotide primers and probes were designed using the nucleotide sequence of DNA1840. A full length plasmid library of cDNAs from human umbilical vein endothelial cells (HUVEC) was titered and approximately 100,000 cfu were plated in 192 pools of 500 cfu/pool into 96-well round bottom plates. The pools were grown overnight at 37°C with shaking (200rpm). PCR was performed on the individual cultures using primers specific to DNA1840. Agarose gel electrophoresis was performed and positive wells were identified by visualization of a band of the expected size. Individual positive clones were obtained by colony lift followed by hybridization with ³²P-labeled oligonucleotide. These clones were characterized by PCR, restriction digest, and southern blot analyses.

A cDNA clone was sequenced in entirety. A nucleotide sequence of DNA19355 is shown in Figure 1 (SEQ ID NO : 2). Clone DNA19355-1150 contains a single open reading frame with an apparent translational initiation site at nucleotide positions 21-23 [Kozak et al., supra] (Fig. 1; SEQ ID NO : 2). The predicted polypeptide precursor is 177 amino acids long and has a calculated molecular weight of approximately 20,308 daltons. Hydropathy analysis suggests a type II transmembrane protein typology, with a putative cytoplasmic region (amino acids 1-25); transmembrane region (amino acids 26-51); and extracellular region (amino acids 52-177). Two potential N-linked glycosylation sites have been identified at position 129 (Asn) and position 161 (Asn) of the sequence shown in Fig. 1 (SEQ ID NO : 1). Clone DNAs19355-1150 has been deposited with ATCC and is assigned ATCC deposit no. 209466. DNA19355 polypeptide is obtained or obtainable by expressing the molecule encoded by the cDNA insert of the deposited ATCC 209466 vector. Digestion of the vector with XbaI and NotI restriction enzymes will yield a 1411 bp fragment and 668 bp fragment.

Based on a BLAST and FastA sequence alignment analysis (using the ALIGN computer program) of extracellular sequence, DNA19355 shows amino acid sequence identity co several members of the TNF cytokine family, and particularly, to human Apo-2L (19.8%), Fas/Apol-ligand (19.0%), TNF-alpha (20.6%) and Lymphotoxin-α (17.5%) (see Figure 2). Most of the amino acid sequence identity is found in the regions corresponding to the beta-strands in the crystal structure of TNF-alpha [Banner et al., Cell, 73:431-435 (1993); Eck et al., J. Biol. Chem., 264:17595-605 (1989); Lewit-Bentley et al., J. Mol. Biol., 199: 389-92 (1988)]. The sequence of strand C is especially conserved in all members of the family (see Figure 2). The sequence between the putative transmembrane domain and the first beta-strand of the DNA19335 polypeptide is relatively short, including 5 residues, as compared to about 30 to about 80 residues in TNF-alpha, CD95L or Apo-2 ligand.

### EXAMPLE 2

### Northern Blot Analysis

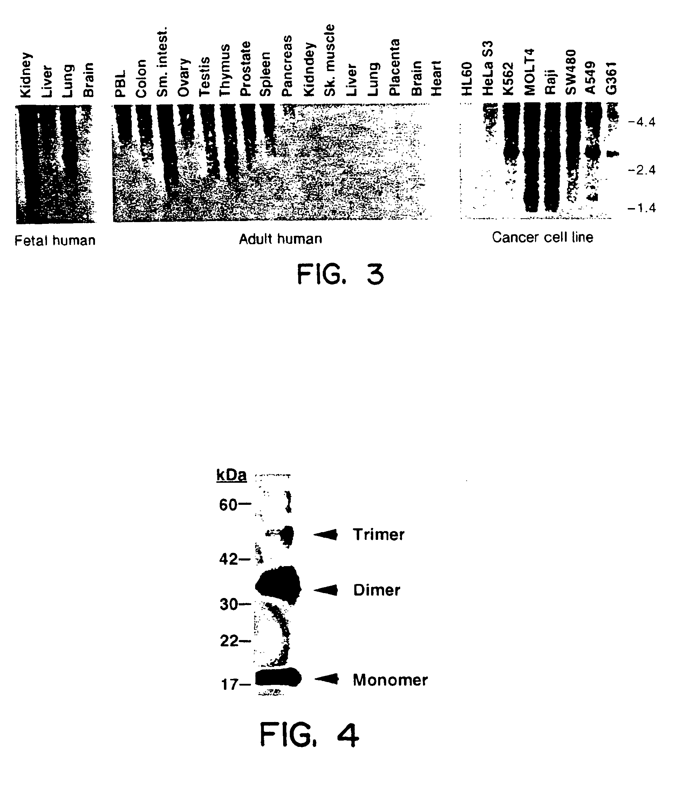

Expression of DNA19355 mRNA in human tissues and tumor cell lines was examined by Northern blot analysis (see Figure 3). Human RNA blots were hybridized to an approximately 700 bp-long ³²P-labeled DNA probe generated by digestion of the pRK5 plasmid encoding full-length DNA19355 cDNA with Xba-I; this probe corresponds to the entire coding sequence plus some flanking 5' and 3' sequences.

Human fetal, adult, or cancer cell line mRNA blots (Clontech) were incubated with the DNA probe in hybridization buffer (5X SSPE; 2X Denhardt's solution; 100 mg/mL denatured sheared salmon sperm DNA; 50% formamide; 2% SDS) for 60 hours at 42°C. The blots were washed several times in 2X SSC; 0.05% SDS for 1 hour at room temperature, followed by a 30 minute wash in 0.1X SSC; 0.1% SDS at 50°C. The blots were developed after overnight exposure by phosphorimager analysis (Fuji).

As shown in Figure 3, a predominant mRNA transcript of about 3.2 kB was detected in fetal kidney and lung, and in adult small intestine. Expression was also detected in 6 out of 8 human tumor cell lines tested, which showed about the same 3.2 kB transcript, as well as weaker expression of about 1.5 and about 5 kB transcripts.

The results indicate that the mRNA expression of the DNA19355 polypeptide is relatively restricted in normal tissues, but is markedly elevated in tumor cell lines from lymphoid as well as non-lymphoid origin.

### EXAMPLE 3

### Expression of DNA19355 in E. coli

The DNA sequence (of Fig. 1; SEQ ID NO : 2) encoding an extracellular region of the DNA19355 polypeptide (amino acids 52 to 177 of Fig. 1; SEQ ID NO : 1) was amplified with PCR primers containing flanking NdeI and XbaI restriction sites, respectively: forward: 5'-GAC GAC AAG CAT ATG TTA GAG ACT GCT AAG GAG CCC TG- 3 ' (SEQ ID NO : 3); reverse: 5'-TAG CAG CCG GAT CCT AGG AGA TGA ATT GGG GATT-3' (SEQ ID NO : 4). The PCR was digested and cloned into the NdeI and XbaI sites of plasmid pET19B (Novagen) downstream and in frame of a Met Gly His₁₀ sequence followed by a 12 amino acid enterokinase cleavage site (derived from the plasmid) : Met Gly His His His His His His His His His His Ser Ser Gly His Ile Asp Asp Asp Asp Lys His Met (SEQ ID NO : 5).

The resulting plasmid was used to transform *E*. *coli* strain JM109 (ATCC 53323) using the methods described in Sambrook et al., supra. Transfor mants were identified by PCR. Plasmid DNA was isolated and confirmed by restriction analysis and DNA sequencing.

Selected clones were grown overnight in liquid culture medium LB supplemented with antibiotics. The overnight culture was subsequently used to inoculate a larger scale culture. The cells were grown to a desired optical density, during which the expression promoter is turned on.

After culturing the cells for several more hours, the cells were harvested by centrifugation. The cell pellet obtained by the centrifugation was solubilized using a microfluidizer in a buffer containing 0.1M Tris, 0.2M NaCl, 50 mM EDTA, pH 8.0. The solubilized DNA19355 protein was purified using Nickel-sepharose affinity chromatography.

The DNA19355 protein was analyzed by SDS-PAGE followed by Western Blot with nickel-conjugated horseradish peroxidase followed by ECL detection (Boehringer Mannheim). Three predominant protein bands were detected, which corresponded in size to monomeric, homodimeric, and homotrimeric forms of the protein (Figure 4). It is believed based on this result that in its native form, in the absence of SDS denaturation, the soluble DNA19355 protein is capable of forming homotrimers.

### EXAMPLE 4

### Apoptotic Activity of DNA19355

The pRK5 plasmid encoding the full-length DNA19355 protein, or empty pRK5 plasmid, or pRK5 encoding full-length human Apo-2 ligand (Apo-2L) was transiently transfected into human 293 cells (10⁶ cells/10 cm dish) by calcium phosphate precipitation. In some cases, the cells were co-transfected with a pRK5 plasmid encoding the poxvirus-derived caspase inhibitor CrmA, or a dominant-negative mutant form of death adaptor protein FADD (FADD-DN), which mediates death signaling by Fas/Apo1 and TNFR1. Sixteen hours later, the cells were stained with Hoechst 33342 dye (10 µg/ml), and apoptotic or normal nuclei were counted under a Leica fluorescence microscope equipped with Hoffmann optics. In some cases, the caspase inhibitor z-VAD-fmk (Research Biochemicals) (200 µM) was added to the dishes immediately after transfection.

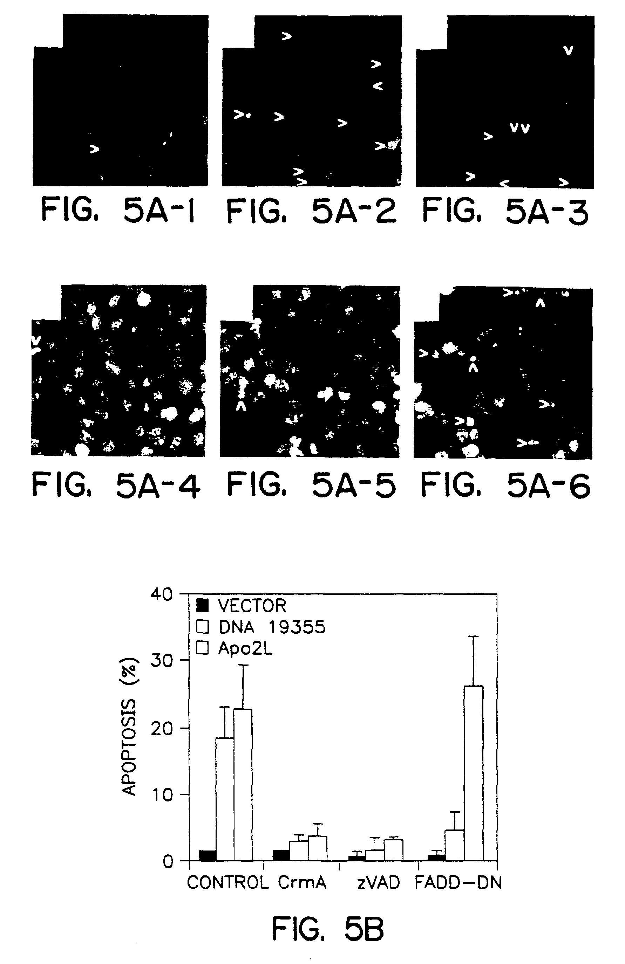

As shown in Fig. 5, transfection by DNA19355 resulted in a substantial increase in the level of apoptosis as compared with pRK5, similar to the increase observed with Apo-2L, a well-established apoptosis inducer. The increase in apoptosis induced by DNA19355 was blocked by CrmA or by z-VAD-fmk, indicating the involvement of caspases in this effect. In addition, the increase in apoptosis induced by DNA19355, but not by Apo-2L, was blocked by FADD-DN, indicating that the FADD adaptor protein plays an essential role in transmitting the death signal from DNA19355 to the caspase machinery.

### EXAMPLE 5

### Activation of NF-κB by DNA19355

The pRK5 plasmid encoding the full-length DNA19355 protein, or empty pRK5 plasmid, or pRK5 encoding full-length human Apo-2L was transiently transfected into human 293 cells (10⁶ cells/10 cm dish) by calcium phosphate precipitation. The cells were co-transfected with empty pRK5 plasmid, or pRK5 plasmid encoding a dominant-negative, kinase deficient, mutant form of the serine/threonine kinase NIK (NIK-DN), which mediates NF-κB activation by TNF [Malinin et al., Nature, 385: 540-544 (1997)]. Sixteen hours later, the cells were harvested, nuclear extracts were prepared, and 1 µg of nuclear protein was reacted with a ³²P-labeled NF-κB-specific synthetic oligonucleotide probe ATCAGGGACTTTCCGCTGGGGACTTTCCG (SEQ ID NO : 6) [see, also, MacKay et al., J. Immunol., 153: 5274-5284 (1994)].

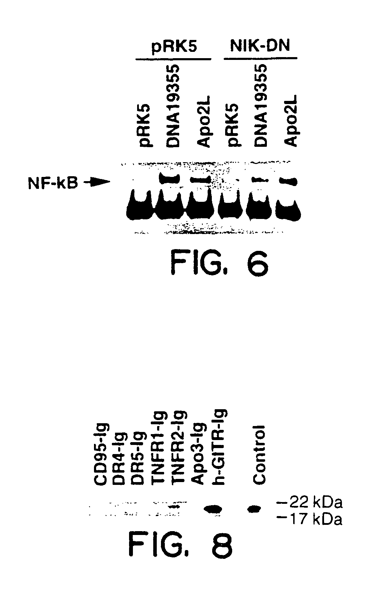

As shown in Fig. 6, transfection by DNA19355 induced significant NF-κB activation, as measured by an electrophoretic mobility shift assay [Marsters et al. PNAS, 92: 5401-5405 (1995)]; the level of activation was greater than the level obtained with Apo-2L. Co-transfection with NIK-DN substantially reduced NF-κB activation by DNA19355, but not activation by Apo-2L. This result suggests that like TNF-alpha, DNA19355 activates NF-κB through a signaling pathway that involves the NIK protein.

### EXAMPLE 6

### Expression of DNA19355 in mammalian cells

This example illustrates preparation of a form of DNA19355 by recombinant expression in mammalian cells.

The vector, pRK5 (see EP 307,247, published March 15, 1989), is employed as the expression vector. Optionally, the DNA19355 DNA is ligated into pRK5 with selected restriction enzymes to allow insertion of the DNA19355 DNA using ligation methods such as described in Sambrook et al., supra. The resulting vector is called pRK5-DNA19355.

In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 µg pRK5-DNA19355 DNA is mixed with about 1 µg DNA encoding the VA RNA gene [Thimmappaya et al., Cell, 31:543 (1982)] and dissolved in 500 µl of 1 mM Tris-HCl, 0.1 mM EDTA, 0.227 M CaCl₂ To this mixture is added, dropwise, 500 µl of 50 mM HEPES (pH 7.35), 280 mM NaCl, 1.5 mM NaPO₄, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 µCi/ml ³⁵S-cysteine and 200 µCi/ml ³⁵S-methionine. After a 12 hour incubation, the conditioned medium is collected, concentrated on a spin filter, and loaded onto a 15% SDS gel. The processed gel may be dried and exposed to film for a selected period of time to reveal the presence of DNA19355 polypeptide. The cultures containing transfected cells may undergo further incubation (in serum free medium) and the medium is tested in selected bioassays.

In an alternative technique, DNA19355 may be introduced into 293 cells transiently using the dextran sulfate method described by Somparyrac et al., Proc. Natl. Acad. Sci., 12: 7575 (1981). 293 cells are grown to maximal density in a spinner flask and 700 µg pRK5-DNA19355 DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 µg/ml bovine insulin and 0.1 µg/ml bovine transferrin. After about four days, the conditioned media is centrifuged and filtered to remove cells and debris. The sample containing expressed DNA19355 can then be concentrated and purified by any selected method, such as dialysis and/or column chromatography.

In another embodiment, DNAs19355 can be expressed in CHO cells. The pRK5-DNA19355 can be transfected into CHO cells using known reagents such as CaPO₄ or DEAE-dextran. As described above, the cell cultures can be incubated, and the medium replaced with culture medium (alone) or medium containing a radiolabel such as ³⁵S-methionine. After determining the presence of DNA19355 polypeptide, the culture medium may be replaced with serum free medium. Preferably, the cultures are incubated for about 6 days, and then the conditioned medium is harvested. The medium containing the expressed DNA19355 can then be concentrated and purified by any selected method.

Epitope-tagged DNA19355 may also be expressed in host CHO cells. The DNA19355 may be subcloned out of the pRK5 vector. The subclone insert can undergo PCR to fuse in frame with a selected epitope tag such as a poly-his tag into a Baculovirus expression vector. The poly-his tagged DNA19355 insert can then be subcloned into a SV40 driven vector containing a selection marker such as DHFR for selection of stable clones. Finally, the CHO cells can be transfected (as described above) with the SV40 driven vector. Labeling may be performed, as described above, to verify expression. The culture medium containing the expressed poly-His tagged DNA19355 can then be concentrated and purified by any selected method, such as by Ni²⁻-chelate affinity chromatography.

### EXAMPLE 7

### Expression of DNA19355 in Yeast

The following method describes recombinant expression of DNA19355 in yeast.

First, yeast expression vectors are constructed for intracellular production or secretion of DNA19355 from the ADH2/GAPDH promoter. DNA encoding DNA19355, a selected signal peptide and the promoter is inserted into suitable restriction enzyme sites in the selected plasmid to direct intracellular expression of DNA19355. For secretion, DNA encoding DNA19355 can be cloned into the selected plasmid, together with DNA encoding the ADH2/GAPDH promoter, the yeast alpha-factor secretory signal/leader sequence, and linker sequences (if needed) for expression of DNA19355.

Yeast cells, such as yeast strain AB110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant DNA19355 can subsequently be isolated and purified by removing the yeast cells from the fermentation medium by centrifugation and then concentrating the medium using selected cartridge filters. The concentrate containing DNA19355 may further be purified using selected column chromatography resins.

### EXAMPLE 8

### Expression of DNA19355 in Baculovirus-infected Insect Cells

The following method describes recombinant expression of DNA19355 in insect cells.

The DNA19355 is fused upstream of an epitope tag contained with a baculovirus expression vector. Such epitope tags include poly-his tags and immunoglobulin tags (like Fc regions of IgG). A variety of plasmids may be employed, including plasmids derived from commercially available plasmids such as pVL1393 (Novagen). Briefly, the DNA19355 or the desired portion of the DNA19355 (such as a sequence encoding an extracellular domain) is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold^{™} virus DNA (Pharmingen) into *Spodoptera frugiperda* ("Sf9") cells (ATCC CRL 1711) using lipofectin (commercially available from GIBCO-BRL). After 4 - 5 days of incubation at 28°C, the released viruses are harvested and used for further amplifications. Viral infection and protein expression is performed as described by O'Reilley et al., Baculovirus expression vectors: A laboratory Manual, Oxford:Oxford University Press (1994).

Expressed poly-his tagged DNA19355 can then be purified, for example, by Ni^{2'}-chelate affinity chromatography as follows. Extracts are prepared from recombinant virus-infected Sf9 cells as described by Rupert et al., Nature, 362:175-179 (1993). Briefly, Sf9 cells are washed, resuspended in sonication buffer (25 mL Hepes, pH 7.9; 12.5 mM MgCl₂; 0.1 mM EDTA; 10% Glycerol; 0.1% NP-40; 0.4 M kCl), and sonicated twice for 20 seconds on ice. The sonicates are cleared by centrifugation, and the supernatant is diluted 50-fold in loading buffer (50 mM phosphate, 300 mM NaCl, 10% Glycerol, pH 7.8) and filtered through a 0.45 µm filter. A Ni²⁺-NTA agarose column (commercially available from Qiagen) is prepared with a bed volume of 5 mL, washed with 25 mL of water and equilibrated with 25 mL of loading buffer. The filtered cell extract is loaded onto the column at 0.5 mL per minute. The column is washed to baseline A₂₈₀ with loading buffer, at which point fraction collection is started. Next, the column is washed with a secondary wash buffer (50 mM phosphate; 300 mM NaCl, 10% Glycerol, pH 6.0), which elutes nonspecifically bound protein. After reaching A₂₈₀ baseline again, the column is developed with a 0 to 500 mM Imidazole gradient in the secondary wash buffer. One mL fractions are collected and analyzed by SDS-PAGE and silver staining or western blot with Ni²-NTA-conjugated to alkaline phosphatase (Qiagen). Fractions containing the eluted His₁₀-tagged DNA19355 are pooled and dialyzed against loading buffer.

Alternatively, purification of the IgG tagged (or Fc tagged) DNA19355 can be performed using known chromatography techniques, including for instance, Protein A or protein G column chromatography.

### EXAMPLE 9

### Preparation of Antibodies that Bind DNA19355

This example illustrates preparation of monoclonal antibodies which can specifically bind DNA19355.

Techniques for producing the monoclonal antibodies are know in the art and are described, for instance, in Goding, supra. Immunogens that may be employed include purified DNA19355, fusion proteins containing DNA19355, and cells expressing recombinant DNA19355 on the cell surface. Selection of the immunogen can be made by the skilled artisan without undue experimentation.

Mice, such as Balb/c, are immunized with the DNA19355 immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect DNA19355 antibodies.

After a suitable antibody titer has been detected, the animals "positive" for antibodies can be injected with a final intravenous injection of DNA19355. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against DNA19355. Determination of "positive" hybridoma cells secreting the desired monoclonal antibodies against DNA19355 is within the skill in the art.

The positive hybridoma cells can be injected intraperitoneally into syngeneic Balb/c mice to produce ascites containing the anti-DNA19355 monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

### EXAMPLE 10

### Use of DNA19355 as a hybridization probe

The following method describes use of a nucleotide sequence encoding DNA19355 as a hybridization probe.

DNA comprising the coding sequence of DNA19355 (as shown in Figure 1, SEQ ID NO : 2) is employed as a probe to screen for homologous DNAs (such as those encoding naturally-occurring variants of DNA19355) in human tissue cDNA libraries or human tissue genomic libraries.

Hybridization and washing of filters containing either library DNAs is performed under the following high stringency conditions. Hybridization of radiolabeled DNA19355-derived probe to the filters is performed in a solution of 50% formamide, 5x SSC, 0.1% SDS, 0.1% sodium pyrophosphate, 50 mM sodium phosphate, pH 6.8, 2x Denhardt's solution, and 10% dextran sulfate at 42°C for 20 hours. Washing of the filters is performed in an aqueous solution of 0.1x SSC and 0.1% SDS at 42°C.

DNAs having a desired sequence identity with the DNA encoding full-length native sequence DNA19355 can then be identified using standard techniques known in the art.

### EXAMPLE 11

### Chromosomal Mapping

Chromosomal localization of the human DNA19355 gene was examined by radiation hybrid (RH) panel analysis. RH mapping was performed by PCR using a mouse-human cell radiation hybrid panel (Research Genetics) and primers based on the coding region of the DNA19355 cDNA [Gelb et al., Hum. Genet., 98:141 (1996)]. Analysis of the PCR data using the Stanford Human Genome Center Database indicated that DNA19355 is linked to the STS marker D1S2790 and to Genethon marker AFMb352xe9, and maps to the human chromosome 1q23. Notably, CD95L also maps to chromosome 1q23 [Takahashi et al., Int. Immunol., 6: 1567-1574 (1994), whereas OX40 ligand maps to chromosome 1q25 [Baum et al., EMBO J., 13: 3992-4001 (1994)]. Accordingly, these TNF family members may have arisen by duplication and divergence of a common ancestral gene.

### EXAMPLE 12

### Binding Specificity of DNA19355 Polypeptide for Human GITR Receptor

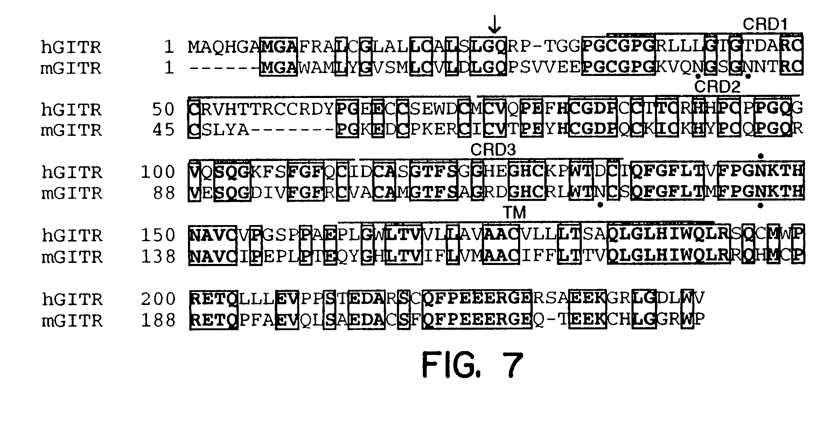

Assays were conducted to determine whether the DNA19355 polypeptide interacts and specifically binds with a human homolog of the receptor molecule referred to as "GITR". A murine GITR (mGITR) polypeptide was described in Nocentini et al., Proc. Natl. Acad. Sci., 94: 6216-6221 (1997). What are believed to be human homologs of the mGITR have been described. An amino acid sequence for a full length human GITR (hGITR) is shown in SEQ ID NO : 4 in PCT WO 98/06842, published February 19, 1998. A comparison of the hGITR and mGITR amino acid sequences is shown in Figure 7.

To test for binding, a soluble immunoglobulin fusion protein (immunoadhesin) which included the hGITR extracellular in (see amino acids 1-167 of Figure 7) was expressed in insect cells. The hGITR ECD was expressed as a C-terminus IgG-Fc tagged form in insect cells using Baculovirus (as described in Example 8 above).

A soluble DNA19355 polypeptide was also prepared by expressing the ECD in E. coli cells (as described in Example 3 above). The soluble DNA19355 ECD molecule was then labeled with ¹²⁵I. For comparison, immunoadhesin constructs were also made of the following TNF receptor family members: CD95, DR4, DR5, TNFR1, TNFR2, and Apo-3. CD95, DR4, DR5, TNFR1, TNFR2, and Apo-3 immunoadhesins were prepared by fusing each receptor's ECD to the hinge and Fc portion of human IgG, as described previously for TNFR1 [Ashkenazi et al., Proc. Natl. Acad. Sci., 88:10535-10539 (1991)]. The respective TNF receptor family members are described (and relevant references cited) in the Background of the Invention section.

For the co-precipitation assay, each immunoadhesin (5 microgram) was incubated with ¹²⁵I-labeled soluble DNA19355 polypeptide (1 microgram) for 1 hour at 24°C, followed by protein A-sepharose for 30 minutes on ice. The reaction mixtures were spun down and washed several times in PBS, boiled in SDS-PAGE buffer containing 20 mM dithiothreitol and then resolved by SDS-PAGE and autoradiography.

The results are shown in Figure 8. The position of the molecular weight markers (kDa) are indicated in the figure. The hGITR-IgG bound to the radioiodinated soluble DNA19355 polypeptide. However, the hGITR-IgG did not bind to the immunoadhesin constructs of CD95, DR4, DR5, TNFR1, TNFR2, or Apo-3.

In another assay, human 293 cells were transiently transfected with DNA19355 and the ability of receptor immunoadhesin constructs for hGITR, TNFR1, HVEM, and DcR1 to bind to those transfected cells was determined by FACS analysis. The 293 cells were maintained in high glucose DMEM media supplemented with 10% fetal bovine serum (FBS), 2mM glutamine, 100 microgram/ml penicillin, and 100 microgram/ml streptomycin. The transfected cells (1 x 10⁵) were incubated for 60 minutes at 4°C in 200 microliters 2% FBS/PBS with 1 microgram of the respective receptor or ligand immunoadhesin. The cells were then washed with 2% FBS/PBS, stained with R-phycoerythrin-conjugated goat anti-human antibody (Jackson Immunoresearch, West Grove, PA). Next, the cells were analyzed by FACS. To test the binding of the respective immunoadhesins to the transiently transfected cells, an expression vector (pRK5-CD4; Smith et al., Science, 328:1704-1707 (1987)) for CD4 was co-transfected with DNA19355 expression vector (see Example 3). FITC-conjugated anti-CD4 (Pharmingen, San Diego, CA) was then used to identify and gate the transfected cell population in the FACS analysis.

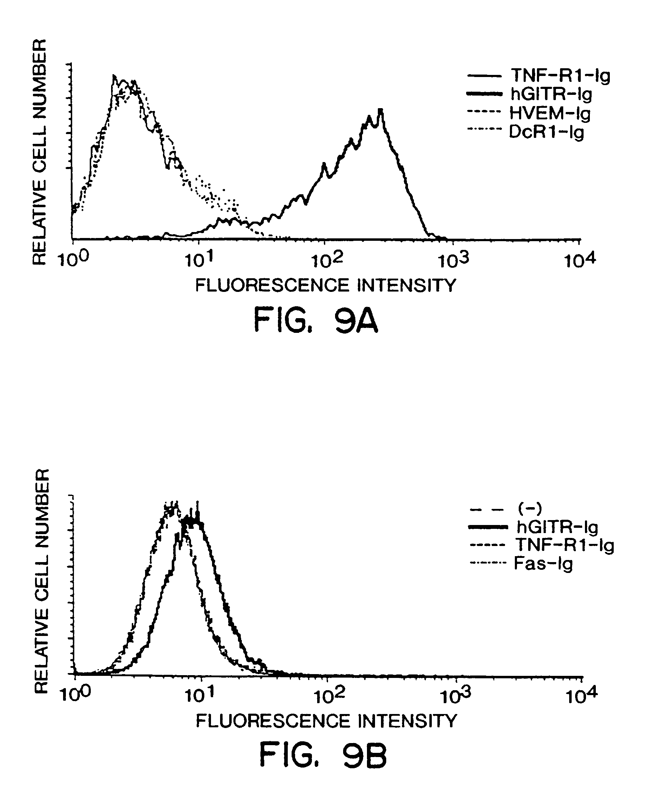

As shown in Figure 9A, the hGITR-IgG bound specifically to the surface of cells transfected with the expression plasmid encoding the full length DNA19355. No such binding was observed for the TNFR1, HVEM or DcR1. The hGITR-IgG did not bind to the cells transfected with a control plasmid (data not shows).

The results demonstrate a specific binding interaction of the DNA19355 polypeptide with hGITR and that the DNA19355 polypeptide does not interact with any of the other TNF receptor family members tested.

The DNA19355 polypeptide was identified in a human umbilical vein endothelial cell (HUVEC) library, and the DNA19355 polypeptide transcripts are readily detectable in HUVEC by RT-PCR (data not shown). A FACS analysis assay was conducted to examine whether specific binding of hGITR-IgG could be demonstrated with HUVEC by FACS analysis. HUVEC cells were purchased from Cell Systems (Kirkland, WA) and grown in a 50:50 mix of Ham's F12 and Low Glucose DMEM media containing 10% fetal bovine serum, 2 mM L-glutamine, 10 mM Hepes, and 10 ng/ml basic FGF. Cells were FACS sorted with PBS, hGITR-IgG, TNFR1-IgG or Fas-IgG as a primary antibody and goat anti-human F(ab')2 conjugated to phycoerythrin (CalTag, Burlingame, CA).

It was found that hGITR-IgG specifically bound to HUVEC. (See Figure 9B). Neither the Fas-IgG nor the TNFR1-IgG exhibited specific binding to the HUVEC cells.

### EXAMPLE 13

### Activation of NF-κB by DNA19355

An assay was conducted to determine whether DNA19355/hGITR induces NF-κB activation by analyzing expression of a reporter gene driven by a promoter containing a NF-κB responsive element from the E-selectin gene.

Human 293 cells (2 x 10⁵) were transiently transfected by calcium phosphate transfection with 0.5 microgram of the firefly luciferase reporter plasmid pGL3.ELAM.tk [Yang et al., Nature, 395:284-288 (1998)] and 0.05 microgram of the Renilla luciferase resporter plasmid (as internal transfection control) (Pharmacia), as well as the indicated additional expression vectors for DNA19355 and hGITR (described above) (0.1 microgram hGITR; 0.5 microgram for other expression vectors), and carrier plasmid pRK5D to maintain constant DNA between transfections. After 24 hours, the cells were harvested and luciferase activity was assayed as recommended by the manufacturer (Pharmacia). Activities were normalized for differences in transfection efficiency by dividing firefly luciferase activity by that of Renilla luciferase and were expressed as activity relative to that seen in the absence of added expression vectors.

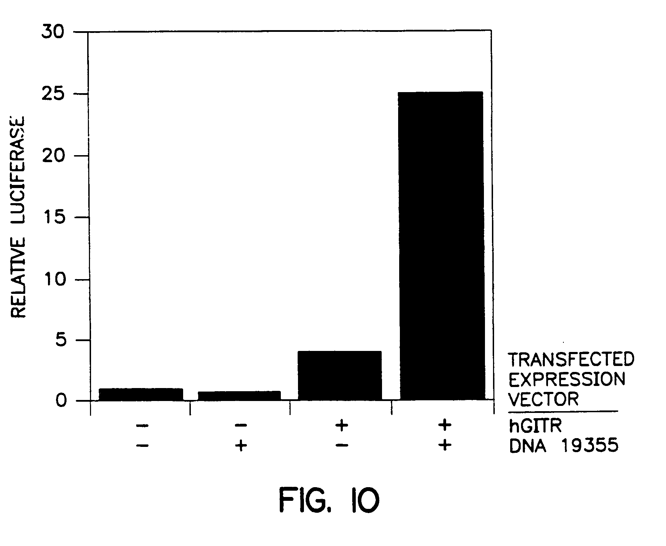

As shown in Figure 10, overexpression of hGITR resulted in significant gene activation, and the observed result was enhanced by co-expression of both DNA19355 and hGITR.

### EXAMPLE 14

### Stimulation of TNF-alpha Production

An assay was conducted to examine production of TNF-alpha and IL-1beta from isolated primary T cells and macrophages in response to stimulation by DNA19355 polypeptide.

Primary T cells or monocyte/macrophages were isolated from human donors. The primary human T cells were isolated from whole blood by a T cell enrichment column (R & D Systems). The monocytes/macrophages were isolated from whole blood by adherence to a tissue culture flask. The respective isolated cells were then treated for 24 hours with the DNA19355 immunoadhesin (see Example 3 above) at 5 microgram/ml in RPMI 1640 medium containing 10% FBS. TNF-alpha levels in the culture supernatants were then determined by ELISA (R & D Systems; according to manufacturer's instructions).

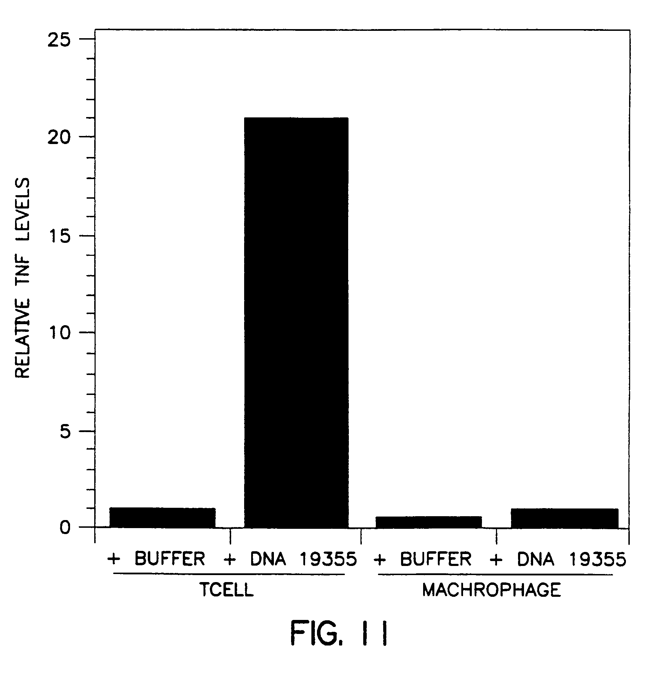

The results are illustrated in Figure 11. The DNA19355 polypeptide induced about a 20-fold increase in secreted TNF-alpha levels from the T cells, but did not affect TNF-alpha release or IL-1beta release from macrophages (data not shown). The induced TNF-alpha production from the human T cells suggests that DNA19355 polypeptide/hGITR contribute to a proinflammatory response.

### EXAMPLE 15

### Guinea Pig Skin Biopsy Assay

An in vivo assay is conducted to determine the activity of a candidate molecule in proinflammatory responses. Specifically, a candidate molecule (such as DNA19355 polypeptide) is injected in guinea pigs and skin biopsies from the treated animal are analyzed for polymorphonuclear/mononuclear cell infiltrate or eosinophil infiltrate.

The guinea pigs are anesthetized with Ketamine (75-80 mg/kg plus 5 mg/kg Xylazine) intramuscularly. The candidate molecule is then injected into skin on the back of the animal at 16 sites (100 microliter per site intradermally). Approximately 1 ml of Evans blue dye/PBS is injected intracordially.

Blemishes at the injection sites are measured (mm diameter) at 1 hour and 6 hours. The guinea pigs are sacrificed at 6 hours after skin injection. Skin samples at the injection sites are excised and fixed in paraformaldehyde. The tissues are then prepared for histological evaluation using standard staining techniques. Analysis of the tissues includes characterizing cell type in the inflammatory infiltrate and evaluating the perivascular infiltrate.

### Deposit of Biological Material

The following materials have been deposited with the American Type Culture Collection, 10801 University Blvd., Manassas, Virginia USA (ATCC):

| | | |
|---|---|---|
| Material | ATCC Dep. No. | Deposit Date |
| DNA19355-1150 | 209466 | November 18, 1997 |

This deposit was made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposit will be made available by ATCC under the terms of the Budapest Treaty, and subject to an agreement between Genentech, Inc. and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC §122 and the Commissioner's rules pursuant thereto (including 37 CFR §1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

The foregoing written specification is considered to be sufficient to enable one skilled in the art to practice the invention. The present invention is not to be limited in scope by the construct deposited, since the deposited embodiment is intended as a single illustration of certain aspects of the invention The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description.

## Claims

1. An isolated polypeptide comprising amino acid residues 1 to 177 of SEQ ID NO: 1.

2. The polypeptide of claim 1 consisting of amino acid residues 1 to 177 of SEQ ID NO: 1.

3. The polypeptide of any one of the preceding claims, wherein the polypeptide binds to human GITR.

4. The polypeptide of any one of the preceding claims encoded by the cDNA insert of the vector deposited as ATCC 209466.

5. An isolated nucleic acid encoding the polypeptide of any one of the preceding claims, wherein the nucleic acid comprises the nucleotide sequence shown as SEQ ID NO: 2 or the cDNA insert of the vector deposited as ATCC 209466.

6. A vector comprising the nucleic acid of claim 5.

7. The vector of claim 6 wherein the nucleic acid is operably linked to control sequences recognized by a host cell transformed with the vector.

8. An isolated host cell comprising the vector of claim 6 or claim 7.

9. The host cell of claim 8, wherein said cell is a CHO cell.

10. The host cell of claim 8, wherein said cell is an E. coli.

11. The host cell of claim 8, wherein said cell is a yeast cell.

12. A process for producing the polypeptide of any one of claims 1 to 4 comprising culturing the host cell of any one of claims 8 to 11 under conditions suitable for expression of the polypeptide and recovering the polypeptide from the culture.

13. A chimeric molecule comprising the polypeptide of any one of claims 1 to 4 fused to a heterologous amino acid sequence.

14. The chimeric molecule of claim 13, wherein said heterologous amino acid sequence is an epitope tag sequence.

15. The chimeric molecule of claim 13, wherein said heterologous amino acid sequence is a Fc region of an immonoglobulin.

16. The chimeric molecule of claim 13, wherein said heterologous amino acid sequence is a leucine zipper.

17. A chimeric molecule comprising the polypeptide of any one of claims 1 to 4 fused to a non-proteinaceous polymer.

## Patentansprüche

1. Isoliertes Polypeptid, umfassend die Aminosäurereste 1 bis 177 aus Seq.-ID Nr. 1.

2. Polypeptid nach Anspruch 1, bestehend aus den Aminosäureresten 1 bis 177 aus Seq.-ID Nr. 1.

3. Polypeptid nach einem der vorangegangenen Ansprüche, worin das Polypeptid an menschlichen GITR bindet.

4. Polypeptid nach einem der vorangegangenen Ansprüche, das von dem cDNA-Insert des Vektors kodiert wird, der als ATCC 209466 hinterlegt ist.

5. Isolierte Nucleinsäure, die für das Polypeptid nach einem der vorangegangenen Ansprüche kodiert, worin die Nucleinsäure die als Seq.-ID Nr. 2 dargestellte Nucleotidsequenz oder das cDNA-Insert des als ATCC 209466 hinterlegten Vektors umfasst.

6. Vektor, umfassend die Nucleinsäure nach Anspruch 5.

7. Vektor nach Anspruch 6, worin die Nucleinsäure operabel an Kontrollsequenzen gebunden ist, die von einer Wirtszelle erkannt werden, die mit dem Vektor transformiert ist.

8. Isolierte Wirtszelle, umfassend den Vektor nach Anspruch 6 oder Anspruch 7.

9. Wirtszelle nach Anspruch 8, worin die Zelle eine CHO-Zelle ist.

10. Wirtszelle nach Anspruch 8, worin die Zelle eine E.-coli-Zelle ist.

11. Wirtszelle nach Anspruch 8, worin die Zelle eine Hefe-Zelle ist.

12. Verfahren zur Produktion des Polypeptids nach einem der Ansprüche 1 bis 4, umfassend das Züchten der Wirtszelle nach einem der Ansprüche 8 bis 11 unter Bedingungen, die sich für die Expression des Polypeptids eignen, sowie das Gewinnen des Polypeptids aus der Kultur.

13. Chimäres Molekül, umfassend das Polypeptid nach einem der Ansprüche 1 bis 4, fusioniert an eine heterologe Aminosäuresequenz.

14. Chimäres Molekül nach Anspruch 13, worin die heterologe Aminosäuresequenz eine Epitopmarkierungssequenz ist.

15. Chimäres Molekül nach Anspruch 13, worin die heterologe Aminosäuresequenz eine Fc-Region eines Immunglobulins ist.

16. Chimäres Molekül nach Anspruch 13, worin die heterologe Aminosäuresequenz ein Leucin-Zipper ist.

17. Chimäres Molekül, umfassend das Polypeptid nach einem der Ansprüche 1 bis 4, fusioniert an ein nicht-proteinisches Polymer.

## Revendications

1. Polypeptide isolé comprenant les résidus d'aminoacides 1 à 177 de la SEQ ID N° 1.

2. Polypeptide suivant la revendication 1, consistant en les résidus d'aminoacides 1 à 177 de la SEQ ID N° 1.

3. Polypeptide suivant l'une quelconque des revendications précédentes, qui se lie au GITR humain.

4. Polypeptide suivant l'une quelconque des revendications précédentes, codé par le segment d'insertion d'ADNc du vecteur déposé sous la référence ATCC 209466.

5. Acide nucléique isolé codant pour le polypeptide de l'une quelconque des revendications précédentes, ledit acide nucléique comprenant la séquence de nucléotides représentée par la SEQ ID N° 2 ou le segment d'insertion d'ADNc du vecteur déposé sous la référence ATCC 209466.

6. Vecteur comprenant l'acide nucléique de la revendication 5.

7. Vecteur suivant la revendication 6, dans lequel l'acide nucléique est lié de manière fonctionnelle à des séquences de régulation reconnues par une cellule hôte transformée avec le vecteur.

8. Cellule hôte isolée comprenant le vecteur de la revendication 6 ou de la revendication 7.

9. Cellule hôte suivant la revendication 8, ladite cellule étant une cellule CHO.

10. Cellule hôte suivant la revendication 8, ladite cellule étant une cellule de E. coli.

11. Cellule hôte suivant la revendication 8, ladite cellule étant une cellule de levure.

12. Procédé pour la production du polypeptide de l'une quelconque des revendications 1 à 4, comprenant la culture de la cellule hôte de l'une quelconque des revendications 8 à 11 dans des conditions convenables pour l'expression du polypeptide et la récupération du polypeptide à partir de la culture.

13. Molécule chimère comprenant le polypeptide de l'une quelconque des revendications 1 à 4 fusionné à une séquence d'aminoacides hétérologue.

14. Molécule chimère suivant la revendication 13, dans laquelle ladite séquence d'aminoacides hétérologue est une séquence de marqueurs épitopiques.

15. Molécule chimère suivant la revendication 13, dans laquelle ladite séquence d'aminoacides hétérologue est une région Fc d'une immunoglobuline.

16. Molécule chimère suivant la revendication 13, dans laquelle ladite séquence d'aminoacides hétérologue est une séquence de fermeture éclair leucine.

17. Molécule chimère comprenant le polypeptide de l'une quelconque des revendications 1 à 4 fusionné à un polymère non protéique.
